# EUROPEAN PATENT SPECIFICATION

(11) **EP 3 233 086 B1**
(45) Date of publication and mention of the grant of the patent: **15.02.2023**
(21) Application number: 15871149.9
(22) Date of filing: 18.12.2015
(51) Int. Cl.: A61K 31/517, C07D 239/94, A61P 41/00, A61L 27/54, A61L 29/16, A61L 31/16

(54) **SUPPRESSION OF NEOINTIMAL FORMATION FOLLOWING VASCULAR SURGERY USING CDK8 INHIBITORS**
UNTERDRÜCKUNG VON NEOINTIMALER FORMUNG NACH VASKULÄRER CHIRURGIE MIT CDK8-INHIBITOREN
SUPPRESSION DE FORMATION NÉO-INTIMALE SUITE À UNE CHIRURGIE VASCULAIRE, À L'AIDE D'INHIBITEURS DE CDK8

(30) Priority: 18.12.2014 US 201462093478 P
(43) Date of publication of application: 25.10.2017
(73) Proprietor: University of South Carolina, Columbia SC 29208 (US)
(72) Inventor: RONINSON, Igor, B., Lexington, SC 29072 (US); CUI, Taixing, Columbia, SC 29212 (US)
(74) Representative: Adam, Holger
(86) International application number: PCT/US2015/066598
(87) International publication number: WO 2016/100782

(56) References cited:
- US-A1- 2007 254 896
- US-A1- 2012 114 722
- US-A1- 2013 217 014
- US-A1- 2014 038 958
- Mario Duran-Prado ET AL: "Cortistatin Inhibits Migration and Proliferation of Human Vascular Smooth Muscle Cells and Decreases Neointimal Formation on Carotid Artery Ligation", Circulation Research, vol. 112, no. 11 24 May 2013 (2013-05-24), pages 1444-1455, XP055490292, DOI: 10.1161/CIRCRESAHA.112.300695 Retrieved from the Internet: URL:http://circres.ahajournals.org [retrieved on 2018-07-05]
- BROOKS, EE ET AL.: 'CVT-313, a Specific and Potent Inhibitor of CDK2 That Prevents Neointima Proliferation.' THE JOURNAL OF BIOLOGICAL CHEMISTRY . vol. 272, no. 46, 14 November 1997, pages 1 - 5, XP002079609 DOI: 10.1074/JBC.272.46.29207
- CHANG, MW ET AL.: 'ADENOVIRUS-MEDIATED OVER-EXPRESSION OF THE CYCLIN/CYCLIN-DEPENDENT KINASE INHIBITOR, P21 INHIBITS VASCULAR SMOOTH MUSCLE CELL PROLIFERATION AND NEOINTIMA FORMATION IN THE RAT CAROTID ARTERY MODEL OF BALLOON ANGIOPLASTY.' JOURNAL OF CLINICAL INVESTIGATION vol. 96, no. 5, 01 November 1995, pages 2260 266 - 2268, XP002296058 DOI: 10.1172/JCI118281

## Description

### BACKGROUND OF THE INVENTION

### Field of the invention

The invention relates to intervention for occlusive vascular disease. More particularly, the invention relates to suppressing neointimal formation resulting from vascular surgery.

### Summary of the related art

Vascular surgery includes many life saving and life prolonging procedures for patients having a variety of vascular diseases. Unfortunately, many of these procedures fail in the medium to long term as a result of neointimal formation, which occludes the area where the surgery was performed.

Many vascular surgical procedures are used to treat occlusive vascular diseases. An important example is coronary artery disease (CAD). Coronary artery bypass vein graft (CABG) surgery remains the most effective surgical treatment for CAD ^{1, 2}. Unfortunately, long-term efficacy of CABG surgery is limited due to vein graft failure. Within 1 year after CABG surgery, 10%~15% of vein grafts occlude, and as many as 50% of vein grafts fail within 10 years and a further 30% have compromised flow because of the neointimal hyperplasia and superimposed atherogenesis ^{1, 2}. Notably, neointimal hyperplasia, a process characterized by abnormal proliferation and accumulation of vascular smooth muscle cells (SMCs), is likely linked to the accelerated graft atherosclerosis ^{1, 2}. Whilst the underlying mechanism of neointimal hyperplasia is yet to be fully understood, to date no therapeutic intervention has proved successful in the treatment of late vein graft failure.

Another vascular surgical procedure for CAD is percutaneous transluminal angioplasty (PTA). Here, again, the initial success of the procedure is overcome as a result of neointimal formation. This is true even when the PTA includes the placement of a stent at the site of the occlusion.

Arterial transplants, both for peripheral and coronary arteries suffer from this same fate. In addition, transplant coronary artery disease (TCAD) remains the most significant cause of morbidity and mortality after orthotopic heart transplantation, and this too involves post-procedural neointimal formation.

Neointimal formation also is the cause of hemodialysis fistula failures. Hemodialysis fistulas are surgically created communications between the native artery and vein in an extremity. Direct communications are called native arteriovenous fistulas. Polytetrafluoroethylene (PTFE) and other materials are used as a communication medium between the artery and vein and are called prosthetic hemodialysis access arteriovenous grafts (AVGs). Both of these can be overcome by neointimal formation. Less than 15% of dialysis fistulas remain patent and functioning without problems during the entire period of a patient's dependence on hemodialysis. This failure is generally treated by PTA, but restenosis remains a problem.

Using vein grafts as an example, it has been documented that the majority of neointimal SMCs in vein grafts are derived from donors; i.e., the vein graft *per se,* and very few originate from the adjacent artery and bone marrow of recipients ^{3, 4, 5, 6} . For many years, a widely accepted view was that, after vein graft implantation, the media SMCs change their phenotype from the quiescent contractile state to the synthetic motile state, a process generally referred to as SMC dedifferentiation, phenotypic modulation, or plasticity ⁷. The synthetic SMCs migrate into intima, expand in number rapidly, and release diverse cytokines and growth factors resulting in neointimal formation. Other documented sources of synthetic SMCs include bone marrow-derived stem cells and resident vascular stem cells (VSCs) ^{8, 9}. However, a recent finding has demonstrated that the differentiation of bone marrow-derived stem cells into vascular SMCs is an exceedingly rare event and the contribution of bone marrow-derived cells to the cellular compartment of the vascular lesion is limited to the transient inflammatory response phase ¹⁰. Therefore, the synthetic SMCs are likely derived from the dedifferentiation of mature vascular SMCs and/or SMC differentiation of resident VSCs. Intriguingly, a recent report indicated the existence of a small population (<10%) of smooth muscle-myosin heavy chain (SM-MHC) negative (⁻) stem cells, named medial-derived multipotent vascular stem cells (MVSCs) in the media of mature blood vessels ¹¹. These cells are negative for the stem cell antigen (Sca)-1 and positive (⁺) for neural crest cell markers such as Sry-box (Sox)10, endoderm marker Sox17, neural cell markers (e.g., neural filament-medium polypeptide (NFM) and glia cell marker S100β), and general mesenchymal stem cell markers including CD29 and CD44. The MVSC activation and differentiation, instead of mature SMC dedifferentiation, was found to result in the synthetic SMCs contributing to vascular lesion formation ¹¹. Even though this "MVSC theory" lacks information regarding the ultimate tracking fate of vascular SMCs via conditional SMC lineage tracing, it has nevertheless challenged the dedifferentiation hypothesis by questioning the previous dogma that vascular disease is a vascular SMC disease and thereby suggesting instead that vascular disease is a stem cell disease ¹². On the other hand, several lines of evidence have also indicated a proatherogenic potential of Sca-1⁺ VSCs in vein grafts ^{13, 14}. However, the relative contributions of dedifferentiation of mature vascular SMCs and of differentiation of resident VSCs to the generation of the synthetic SMCs leading to neointimal hyperplasia in vein grafts remain unknown.

Clinical trials have revealed that statins limit the progression of atherosclerosis in saphenous vein grafts and reduce postoperative cardiovascular events, presumably due to their pleiotropic effects such as inhibition of SMC proliferation and migration ^{2, 15}. Of interest, a recent report documented that peri-adventitial delivery of pravastatin via Pluronic F-127 gel immediately after the transplantation reduced neointimal hyperplasia up to 4 weeks in vein grafts ¹⁶. However, drug release from Pluronic F-127 gel could only be locally maintained during the initial 3 days after transplantation ¹⁷, when a substantial amount of cells in vein grafts may die via apoptosis or necrosis ^{18, 19},

Statins are at present the only drugs shown to diminish the graft failure rate after CABG surgery, but their effects are only partial and graft vein occlusion still occurs even after high-dose statin treatment (Shah et al., Journal of the American College of Cardiology, 51, 1938 - 1943, 2008). Statins also show a wide range of toxicities, such as muscle aches, myositis, difficulty sleeping, dizziness, depression and diabetes (Hu et al., Ther Adv Drug Saf 3, 133-144, 2012; Forcillo et al., Canadian Journal of Cardiology 28, 623-625, 2012; Moßhammeret al., Br J Clin Pharmacol. 78, 454-466, 2014). Hence, there is a need for an alternative class of drugs, acting through a different mechanism than statins, which can be used to reduce post-procedure neointimal formation, either instead of or in combination with statins.

Mario Duran-Prado et al. relates to Cortistatin that inhibits migration and proliferation of human vascular smooth muscle cells and decreases neointimal formation on carotid artery ligation (see Circulation Research, vol. 112, no. 11, 24 May 2013, pages 1444-1455).

US 2007/254896 A1 is directed to substituted pyrimidine compounds and use for treating a chronic or acute protein kinase mediated disease, disorder or condition.

Brooks, E. E. et al. relates to CVT-313, a specific and potent inhibitor of CDK2 that prevents neointima proliferation (see The Journal of Biological Chemistry, vol. 272, no. 46, 14 November 1997, pages 1-5).

Chang, M. W. et al. relates to adenovirus-mediated over-expression of the cyclin/cyclin-dependent kinase inhibitor (see Journal of Clinical Investigation., vol. 96, no. 5, November 1, 1995, pages 2266-2268).

### BRIEF SUMMARY OF THE INVENTION

The invention relates to one or more inhibitors of CDK8/19 for use in suppressing neointimal formation resulting from vascular surgery.

In some embodiments, the vascular surgery is the implantation of a vein graft. In some embodiments the vein graft is implanted in the patient via coronary artery bypass graft (CABG) surgery. In some embodiments the vein graft is implanted in the patient via carotid artery bypass graft surgery. In some embodiments, the vascular surgery is percutaneous transluminal angioplasty (PTA), which may be conducted with or without stent emplacement. In some embodiments the vascular surgery is the creation of arteriovenous fistulae, the preferred access for hemodialysis, which may be native or prosthetic arteriovenous fistulae. In some embodiments the vascular surgery is arterial transplantation which may involve peripheral or coronary arteries. In some embodiments the vascular surgery is orthotopic heart transplantation.

Mechanistically, the present inventors have discovered that the suppression of resident vascular stem cell (VSC) proliferation and SMC differentiation, rather than the inhibition of SMC proliferation, is a primary mechanism by which simvastatin suppresses neointimal formation in vein grafts. In addition, inhibitors of cyclin-dependent kinase 8 (CDK8), a mediator of the stem cell phenotype and of TGFβ signaling that plays a key role in VSC differentiation, did not affect vascular SMC proliferation but suppressed VSC proliferation and SMC differentiation, and inhibited neointimal formation in vein grafts. Our findings reveal an essential role of resident VSCs in the neointimal hyperplasia of vein grafts and demonstrate the therapeutic efficacy of targeting resident VSCs by CDK8 inhibitors in suppressing neointimal hyperplasia of vein grafts. These findings extend to all types of neointimal hyperplasia, indicating that selectively targeting resident VSCs appears to be a novel avenue for the treatment of neointimal formation resulting from vascular surgery.

Further embodiments of the invention are disclosed in the claims.

### BRIEF DESCRIPTION OF THE DRAWINGS

Fig. 1A shows representative immunofluorescence staining of SMA, Smoothelin, SM-MHC, and TUNEL. SMA was labeled with orange (panels a to f), smoothelin with green (panels g to l), SM-MHC with purple (panels m to r), and TUNEL with red (panels s to x). Nuclei were labeled with DAPI (blue).
Fig. 2A shows representative immunofluorescence staining of Sca-1, Sox 17, NFM, and Ki67. Sca-1 was labeled with green (panels a to f), Sox17 with red (panels g to l), NFM with orange (panels m to r), and Ki67with red (panels s to x). The nuclei were labeled with DAPI (blue).
Fig. 3 shows results of tri-immunofluorescence staining of Sca-1, SMA, and Ki67 in cross-sections of jugular vein isografts on day 0, day 7, and day 14 after transplantation. Fig. 3A shows quantified proportions of the cells double positive with Sca-1 and Ki67, Sca-1 and SMA, and SMA and Ki67 in the adventitia and neointima. Figs. 3B-D show representative immunofluorescence staining of Sca-1 (green), SMA (orange), and Ki67 (red). Nuclei were labeled with DAPI (blue).
Fig. 4A shows the effect of the local simvastatin on neointimal formation 4 weeks after transplantation. Fig. 4B shows quantified lumen areas, neointima (NI) areas, NI thickness, and SMA positive areas and thickness. Fig 4C shows the effect of local simvastatin on Sca-1⁺ cell proliferation and SMC differentiation at 3 and 7 days. Fig. 4D shows the effect of simvastatin on Sca-1⁺ cell growth.
Fig. 5 shows the effect of peri-vascular delivery of Senexin A on Sca-1⁺ cell proliferation and SMC differentiation as well as neointimal formation in the jugular vein isografts. Fig. 5A shows the effect of Senexin A on Sca-1⁺ cell growth, with a growth curve of mouse Sca-1⁺ cells cultured in stem cell growth medium with or without Senexin A. Fig. 5B shows the effect of Senexin A on RASMC proliferation. Fig. 5C shows the effect of local Senexin A on neointimal formation 4 weeks after transplantation. Fig. 5D shows the effect of local Senexin A on SMC differentiation at 7 days.
Fig. 6 shows the effect of Senexin B treatment on neointimal formation and Sca-1⁺ VSC differentiation into SMCs in jugular vein isografts. Fig. 6A shows, Left panel; representative H&E staining (upper) and SMA staining (lower); Right panel; quantified lumen areas, neointima (NI) areas, NI thickness, and SMA positive areas and thickness. Fig. 6B shows representative immunofluorescence staining for SMA and Sca-1 on jugular vein isograft cross-sections of vehicle and Senexin B-treated groups (n=4) at 14 and 28 days after transplantation.
Fig. 7A shows representative H&E staining of mouse normal control jugular veins and jugular vein grafts 6 wks after transplantation. Fig. 7B shows representative SMA immunofluorescence staining of mouse normal control jugular veins and jugular vein grafts 6 wks after transplantation. Fig. 7C shows the method of serial sectioning. Fig. 7D shows a scheme to depict structure of a vein graft.
Fig. 8A shows morphological analysis of serial H&E stained cross sections of 5-mm vein grafts from the proximal to distal end. Fig. 8B shows morphological analysis of serial SMA stained cross sections of 5-mm vein grafts from the proximal to distal end.
Fig. 9A shows representative H&E staining of vein cross sections at 0 day and 1, 3, 7, 14, 42 days after the transplantation. Fig. 9B shows schemes depicting main structures of vein grafts at different stages after the transplantation.
Fig. 10 shows representative double-immunofluorescence staining for SMA and VSCs markers Sca-1, Sox17, or NFM on tissue cross-sections of vein grafts (n=4) at 7 and 14 days after transplantation.
Fig. 11 shows representative double-immunofluorescence staining for Ki67 and Sca-1 or SMA on tissue cross-sections of vein grafts (n=4) at 7 and 14 days after transplantation.
Fig. 12 shows the effect of simvastatin treatment on neointimal formation in jugular vein isografts 4 wks after transplantation. Simvastatin (1.6 mg/kg/d) was administrated by intragastric gavage; (Figs. 12A and B) 3 days before transplantation or (Figs. 12C and D) 3 days after transplantation.
Figure 13 shows the effect of perivascular simvastatin treatment on Sca-1⁺ VSC proliferation and SMC differentiation in jugular vein isografts.
Fig. 14 shows the effect of peri-vascular simvastatin treatment on NFM⁺ and Sox17⁺ VSC differentiation into SMCs in jugular vein isografts.
Fig. 15 shows the effect of Senexin B treatment on neointimal formation in jugular vein isografts 4 wks after transplantation.
Fig. 16 shows the effect of a short-term peri-vascular Senexin A treatment on neointimal formation in jugular vein isografts 100 days after transplantation

### DETAILED DESCRIPTION OF THE PREFERRED EMBODIMENTS

The invention relates to one or more inhibitors of CDK8/19 for use in suppressing neointimal formation resulting from vascular surgery.

In some embodiments, the vascular surgery is implantation of a vein graft. Thus, the invention relates to the use of one or more inhibitors of CDK8/19 for suppressing neointimal formation of vein grafts resulting from intervention for occlusive vascular disease, comprising administering to a patient receiving a vein graft. In some embodiments the vein graft is implanted in the patient via coronary artery bypass graft (CABG) surgery. In some embodiments the vein graft is implanted in the patient via carotid artery bypass graft surgery.

In some embodiments, the vascular surgery is percutaneous transluminal angioplasty (PTA), which may or may not include stent emplacement. In some embodiments the vascular surgery is the creation of arteriovenous fistulae, the preferred access for hemodialysis, which may be native or prosthetic arteriovenous fistulae. In some embodiments the vascular surgery is arterial transplantation which may involve peripheral or coronary arteries. In some embodiments the vascular surgery is orthotopic heart transplantation, and suppression of neointimal formation is needed to prevent transplant coronary artery disease (TCAD), the most significant cause of morbidity and mortality after orthotopic heart transplantation.

For purposes of the invention, an "inhibitor of CDK8/19" is a small molecule that inhibits the activity of CDK8, CDK19, or both, to a greater extent than it inhibits another CDK, e.g., CDK9. In preferred embodiments, such greater extent is at least 2-fold more than CDK9. A "small molecule compound" is a molecule having a formula weight of about 800 Daltons or less.

CDK8/19 has become an actively pursued target for drug discovery and development (Rzymski et al., 2015). Accordingly, CDK8/19 inhibitors have become well known in the art. Some of the published CDK8/19 inhibitors, aside from Senexin A (Porter et al., 2012) and Senexin B (US20140038958) include Cortistatin A (which, in addition to CDK8/19 also inhibits Rock kinases) (Cee et al., 2009), CCT251545 (Dale et al., 2015) and SEL120-34A (Rzymski et al., 2015). Any of these, as well as any newly discovered small molecule CDK8/19 inhibitors can be used in the invention.

Depending on the procedure, in some embodiments, the one or more inhibitors of CDK8/19 is applied on a stent, or on a biomaterial (Knight et al., Front. Mater., 10 June 2014 | doi: 10.3389/fmats.2014.00004). In some embodiments, the one or more inhibitors of CDK8/19 is applied to a vein or artery graft, or to a prosthetic hemodialysis access arteriovenous graft perivascularly or intravascularly. In some embodiments, the one or more inhibitors of CDK8/19 is applied to a vein or artery graft, or to a prosthetic hemodialysis access arteriovenous graft ex vivo, by soaking the vein graft in an inhibitor-containing solution prior to transplantation.

The one or more inhibitors of CDK8/19 can be incorporated into a biodissolvable polymer, such as polyvinyl alcohol or polyethylene glycol, which is used to coat a dilatation balloon. (See, e.g., Scott et al., Eur J Pharm Biopharm. 2013 May;84(1): 125-31.) Alternatively, a metallic stent can be coated with silicon based microprobes to deliver the one or more inhibitors of CDK8/19 through the internal elastic lamina and into the compressed atherosclerotic plaque. (See, e.g., Reed et al. (1998) J Pharmaceutical Sci. 87: 1387-1394.) The one or more inhibitors of CDK8/19 can also be mixed with biodegradable poly(lactic-co-glycolic acid) and used to coat a stent. (See, e.g., Zhu et al., (2014) J. Biomechanical Engineering 136: 111004-1-111004-10.) Electrospinning and electrospraying techniques can also be used to coat the stent with nanofibers containing the one or more inhibitors of CDK8/19. (See, e.g., Zamani et al., (2013) Int. J. Nanomedicine 8: 2997-3017; Song et al., J Biomed Mater Res Part B (2012) 100B:2178-2186; Sill et al. (2008) Biomaterials 29: 1989-2006.)

The one or more inhibitors of CDK8/19 can also be administered to the patient systemically, parenterally, or orally. In some embodiments, the one or more inhibitor of CDK8/19 is administered to the patient starting one or more day prior to surgery. In some embodiments, the one or more inhibitors of CDK8/19 is administered to the patient starting immediately after surgery.

In some embodiments, the one or more inhibitors of CDK8/19 is administered in combination with a statin. "In combination with" generally means administering a specific CDK8/19 inhibitor and statin in the course of treating a patient. Such administration may be done in any order, including simultaneous administration, as well as temporally spaced order from a few seconds up to several days apart. Such combination treatment may also include more than a single administration of the specific CDK8/19 inhibitor and statin. The administration of the specific CDK8/19 inhibitor and statin may be by the same or different routes.

For the use according to the invention, the specific CDK8/19 inhibitor may be incorporated into a pharmaceutical formulation. Such formulations comprise the CDK8/19 inhibitor, which may be in the form of a free acid, salt or prodrug, in a pharmaceutically acceptable diluent (including, without limitation, water), carrier, or excipient. Such formulations are well known in the art and are described, e.g., in Remington's Pharmaceutical Sciences, 18th Edition, ed. A. Gennaro, Mack Publishing Co., Easton, Pa., 1990. The characteristics of the carrier will depend on the route of administration. As used herein, the term "pharmaceutically acceptable" means a non-toxic material that is compatible with a biological system such as a cell, cell culture, tissue, or organism, and that does not interfere with the effectiveness or the biological activity of the specific CDK8/19 inhibitor(s). Thus, compositions according to the disclosure may contain, in addition to the inhibitor, diluents, fillers, salts, buffers, stabilizers, solubilizers, and other materials well known in the art. As used herein, the term pharmaceutically acceptable salts refers to salts that retain the desired biological activity of the specific CDK8/19 inhibitor and exhibit minimal or no undesired toxicological effects. Examples of such salts include, but are not limited to, salts formed with inorganic acids (for example, hydrochloric acid, hydrobromic acid, sulfuric acid, phosphoric acid, nitric acid, and the like), and salts formed with organic acids such as acetic acid, oxalic acid, tartaric acid, succinic acid, malic acid, ascorbic acid, benzoic acid, tannic acid, palmoic acid, alginic acid, polyglutamic acid, naphthalenesulfonic acid, naphthalenedisulfonic acid, methanesulfonic acid, p-toluenesulfonic acid and polygalacturonic acid. The specific CDK8/19 inhibitor can also be administered as pharmaceutically acceptable quaternary salt known by those skilled in the art, which specifically include the quaternary ammonium salt of the formula -- NR+Z--, wherein R is hydrogen, alkyl, or benzyl, and Z is a counterion, including chloride, bromide, iodide, -- O-alkyl, toluenesulfonate, methylsulfonate, sulfonate, phosphate, or carboxylate (such as benzoate, succinate, acetate, glycolate, maleate, malate, citrate, tartrate, ascorbate, benzoate, cinnamoate, mandeloate, benzyloate, and diphenylacetate). The specific CDK8/19 inhibitor is included in the pharmaceutically acceptable carrier or diluent in an amount sufficient to deliver to a patient a therapeutically effective amount without causing serious toxic effects in the patient treated. A "therapeutically effective amount" is an amount sufficient to suppress neointima formation. The effective dosage range of the pharmaceutically acceptable derivatives can be calculated based on the weight of the parent compound to be delivered. If the derivative exhibits activity in itself, the effective dosage can be estimated as above using the weight of the derivative, or by other means known to those skilled in the art. The dose in each patient may be adjusted depending on the clinical response to the administration of specific CDK8/19 inhibitor or of the specific CDK8/19 inhibitor and statin.

The present inventors determined the fate of smooth muscle cells (SMCs) and vascular stem cells (VSCs) during the neointimal formation in isologously transplanted jugular veins, and observed that SMCs of vein isografts died within 3 days after transplantation whereas resident VSCs including Sca-1⁺, Sox17⁺, and NFM⁺ cells survived, proliferated and differentiated into SMCs, subsequently associated with neointimal hyperplasia. Of interest, Sca-1⁺ VSCs appear to be the major source of synthetic SMCs. By comparing the therapeutic effect on neointimal hyperplasia of oral administration of simvastatin 3 days before and after the transplantation as well as a peri-vascular delivery of simvastatin during 3 days immediate after the transplantation, we surprisingly found that inhibiting the early stage activation of VSCs is most likely the primary mechanism by which statin suppresses neointimal hyperplasia in vein grafts. Moreover, simvastatin dramatically inhibited Sca-1⁺ cell proliferation in vitro.

We have also found that Senexin A and B, specific inhibitors of CDK8/19 ²⁰, a transcription-regulating kinase that plays a role in stem cell differentiation ²¹ and TGF-β signaling ²² (a mediator of VSC differentiation) preferentially suppress Sca-1⁺ cell proliferation and differentiation into SMCs, and inhibit neointimal hyperplasia in transplanted veins. Collectively, we demonstrated for the first time that statins inhibit neointimal hyperplasia in vein grafts primarily via inactivation of VSCs, and selective inactivation of VSCs by CDK8 inhibitors suppresses neointimal hyperplasia in vein grafts. These results suggest an essential role of VSCs in the pathogenesis of vein graft failure and targeting VSCs represents a novel venue for the treatment of late vein graft failure.

We determined that SMC death coincides with VSC survival and proliferation at an early stage of neointimal hyperplasia in vein grafts, and VSC differentiation into SMCs is associated with the progression of neointimal hyperplasia in vein grafts. Several animal models of vein graft hyperplasia have been successfully established by engrafting veins into arteries using either sutures or cuffs ²³. Of note, a murine model of vein graft hyperplasia using a polyethylene cuff, which was originally described by Zou et al. ²⁴, appears to be most feasible to quantify and obtain reproducible results ^{25, 26}. In this model, i.e., when external jugular vein or vena cava were isografted into carotid arteries of C57BL/6J mice, marked loss of SMCs and dramatic increase in apoptosis in vein grafts were observed 1 week after transplantation. Massive infiltration of inflammatory cells in vein grafts was observed between 2 to 4 weeks and a significant proliferation of SMC to constitute neointimal lesion was evidenced 4 weeks after transplantation. A recent study showed that Sca-1⁺ cells in adventitia in vein grafts were increased 1 week after transplantation ¹⁴. However, the cellular events during the first week in vein grafts after transplantation as well as the cellular dynamics regarding the fate of SMCs and VSCs remained to be fully characterized. Therefore, we adapted the cuff model of external jugular vein isografts and performed a detailed time-course study with endpoints of 0, 1, 3, 7, 14, 28, and 42 days after transplantation.

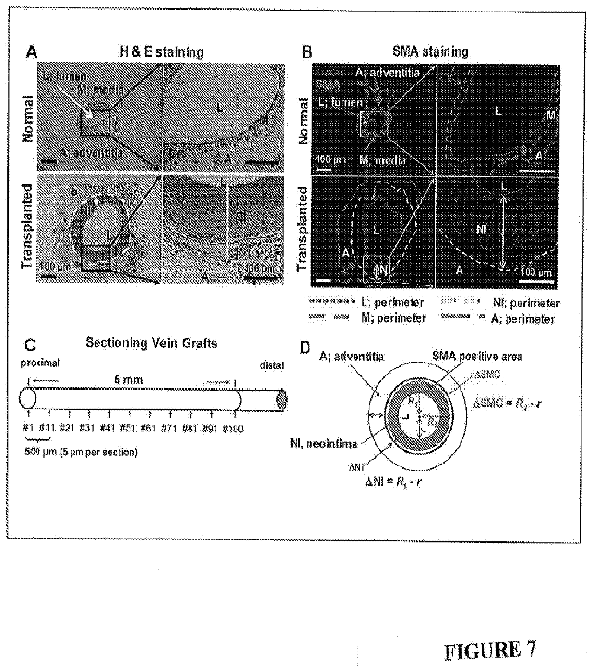

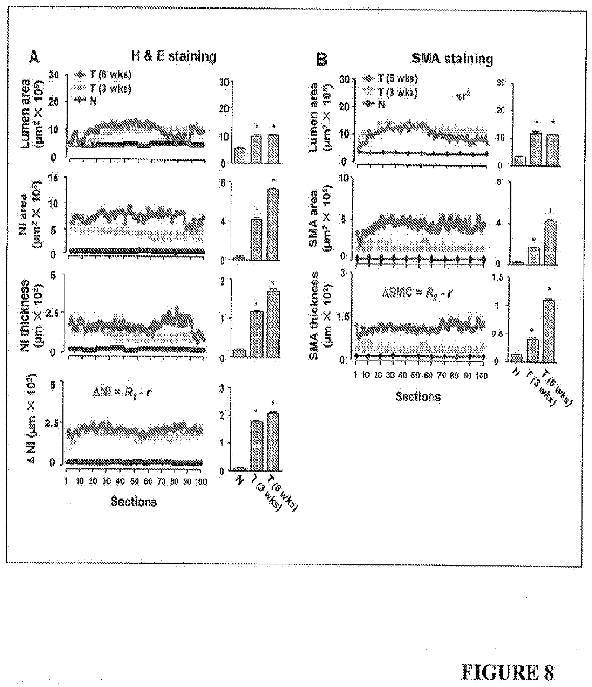

A well accepted method to quantify neointimal lesion is to calculate the thickness of vein graft by measuring 4 regions of a section along a cross and recorded in micrometers ²⁴. We observed clear neointimal formation in vein grafts 42 days after transplantation (Fig. 7A). Immunofluorescent staining revealed that the majority of neointimal cells were smooth muscle alpha actin (SMA)⁺ cells (Fig. 7B), presumably the same synthetic SMCs as described elsewhere ¹⁰. However, we found that there was a gap between decentered borders of neointima and SMA⁺ areas (Fig. 7B). Thus, we further quantified SMC areas in vein grafts via automatic measurement of SMA positive area under an immunofluorescent microscope via the Volocity software (PerkinElmer, Waltham, MA, USA). We compared the neointimal area measured by H&E staining and SMC area by immunofluorescent SMA staining. To reach a solid conclusion, we analyzed 100 sections obtained by selecting the first of every 10 sections from each vein graft, reflecting the magnitude of neointimal formation as well as the amount of neointimal SMCs in a 5-mm vein graft segment (Fig. 7C). As shown in Figure 8, the ratios of neointimal area and thickness as well as SMA⁺ area and thickness were well correlated, showing a similar tendency. Therefore, we determined neointimal area and thickness, and lumen area by H&E staining while quantifying the amount of neointimal SMCs and the thickness of SMC layer by the SMA staining. Consequently, we could examine the contribution of SMCs to neointimal formation and lumen patency in vein grafts.

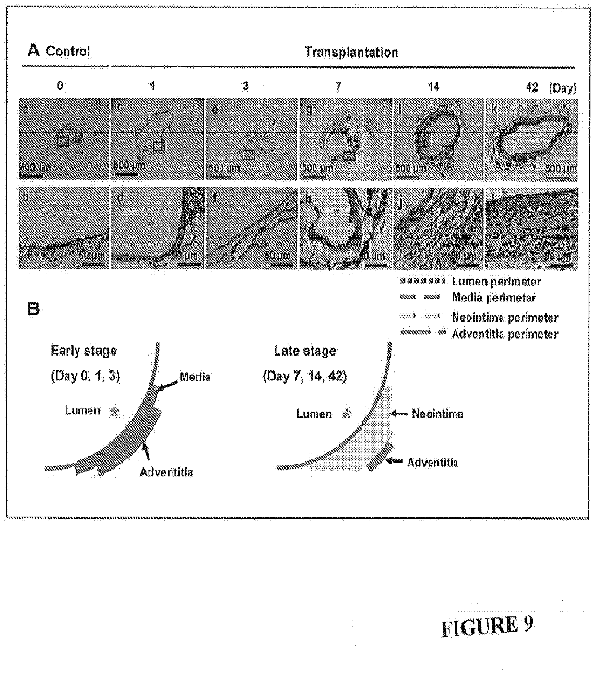

We discovered that SMCs die while VSCs survive and proliferate in vein isografts during the first 3 days after transplantation, and VSC differentiation into SMCs is associated with the progression of neointimal hyperplasia in vein grafts. The normal external jugular veins (day 0) as well as the vein isografts at 1, 3, 7, 14, and 42 days after transplantation were harvested and sectioned for H&E staining and immunofluorescent staining of SMC, VSC, apoptosis, and proliferation markers, respectively. In the normal external jugular vein, H&E staining revealed that two layers of cells, a monolayer each of endothelial and smooth muscle cells, formed the intima and media, whereas the adventitia was composed of connective tissues, including vasa vasorum (Fig. 9A, panels a and b). The thickness of normal jugular vein was approximately 10 to 20 µm (Fig. 9A, panels a and b). However, significant cell loss in the intima and media of vein grafts started on day 1 and peaked at 3 days (Fig. 9A, panels c to f). Notably, it was hard to identify intact nuclei in the intima and media on day 3 (Fig. 9A, panel f). Thereafter, the decellularized vessel walls were recellularized, signs of recellularization appearing from day 7, and subsequently developed neointimal hyperplasia (Fig. 9A, panels g to 1). Thus, there are at least two stages in the vein graft remodeling; i.e., an early stage (0-3 days) characterized by dramatic cell loss in intima and media of vein grafts and a late stage (7-42 days) initiating with recellularization of the vessel wall and followed by neointimal hyperplasia. These results are consistent with previous findings of vein graft remodeling in mice ^{18, 24} and rats ¹⁹, although the dynamics of cellular events are slightly different.

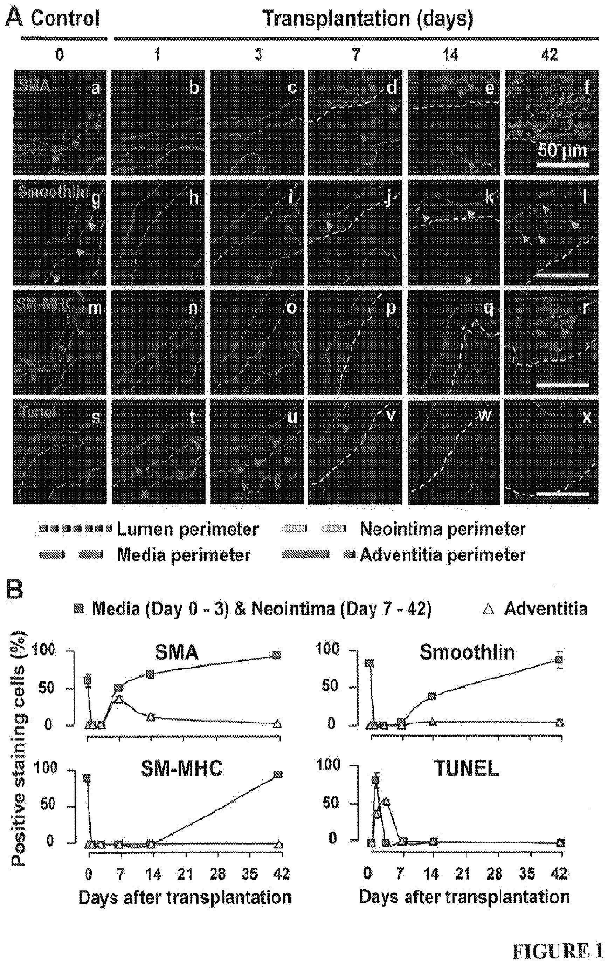

We then analyzed the cellular events in vein isografts regarding the contributions of SMC death, proliferation, and dedifferentiation to neointimal hyperplasia via immunofluorescent microscopic analysis. SMCs were identified by staining smooth muscle contractile proteins including smooth muscle actin alpha (SMA), smoothelin, and smooth muscle myosin heavy chain (SM-MHC). Cell death was assessed by TUNEL staining. A monolayer of SMCs was visualized by strong positive staining of these three contractile proteins in the normal jugular veins (Fig. 1A, panels a, -g, and -m). While the SMC marker staining was negative in the vein grafts within 3 days after transplantation (Fig. 1A, panels b, -c, h, -i, -n, and -o), rare nuclear fragments remaining in the media area were stained positively by TUNEL (Fig. 1A, panels -t and -u between the red and green dotted line). These results suggest that most mature SMCs in the media of the transplanted veins died within 3 days after the transplantation, most likely due to apoptosis and/or necrosis as previously described ^{18, 19}. When the decellularized media were recellularized at 7 days (Fig. 9A, panels -g and -h), some of these cells were positive for SMA and smoothelin staining (Fig. 1A, panels -d and j). The number of SMA and smoothelin positive cells (Fig. 1A, panels -e and k) increased at 14 days, and the SM-MHC positive cells additionally emerged at 42 days (Fig. 1A, panels -f, -l, and -r). Neointimal hyperplasia in vein grafts likely started at 7 days and progressed over time after transplantation (Fig. 1A, panels -d to f, -j to l, and -p to -r, and Fig. 9A, panels -h, -j, and -l). The proportions of positive cells for each contractile protein markers of SMCs and TUNEL staining in media/neointima and adventitia are shown in Fig. 1B and summarized in Table 1. Collectively, these results demonstrate that mature SMCs die at the early stage (0-3 days) after transplantation in vein grafts, and SMCs reappear and proliferate at the late stage (7-42 days), which is associated with the progression of neointima formation. Importantly, these results also indicate that the newly emerged SMCs are not likely derived from the dedifferentiation of mature media SMCs as previously proposed ⁷, but from other sources such as VSCs ^{12, 13, 14}.

**Table 1. Dynamics of SMC and VSC death and proliferation in media, neointima, and adventitia.**

| Graft age (day) | (Total nuclei) | % (total cell number)/5 mm | | | | | | | |
|---|---|---|---|---|---|---|---|---|---|
| | | SMA | Smoothelin | SM-HMC | Tunel | Sca-1 | Sox17 | NFM | Ki67 |
| 0 | Med or Neo Adv | 58.3 ± 8.8 (345) | 79.614.0 (469) | 84.8 ± 4.2 (411) | 0 (369) | 0 (345) | 38.4± 4.1 (495) | 1.4 ± 1.2 (348) | 0 (346) |
| | | 0 (222) | 0 (159) | 0 (147) | 0 (261) | 23.7 ± 6.4 (234) | 6.8 ± 10 (186) | 1.1±1.9 (237) | 0 (222) |
| 1 | Med or Neo Adv | 0^{a} (12) | 0^{a} (42) | 0^{a} (66) | 80.5 ± 9.3' (81) | 0 (36) | 0^{a} (61) | 0^{a} (48) | 0 (27) |
| | | 0.0 ± 0.0 (132) | 0.0 ± 0.0 (204) | 0.0 + 0.0 (204) | 36.4 ± 6.2^{a} (306) | 18.2 ± 5.3^{a} (282) | 14.3 + 3.3 (204) | 5.7 ± 1.0^{a} (204) | 8.3 ± 4.4^{a} (150) |
| 3 | Med or Neo Adv | 0^{a} (0) | 0^{a} (0) | 0^{a} (0) | 0 (0) | 0 (0) | 0^{a} (0) | 0^{a} (0) | 0 (0) |
| | | 0 (684) | 0 (501) | 0 (501) | 62.3 ± 3.8^{a} (213) | 20.6 + 5.3^{a} (816) | 11.9 ± 6.0^{a} (531) | 11.9 ± 6.0^{a} (531) | 13.8 ± 2.3^{a} (192) |
| 7 | Med or Neo Adv | 49.0 ± 2.0^{a} (624) | 4.2 ± 1.3^{a} (408) | 0^{a} (408) | 3.2 ± 1.6^{a} (604) | 31.0 ± 3.6^{a} (447) | 24.6 ± 3.4^{a} (423) | 31.2 ± 7.4^{a} (489) | 14.1 +0.3^{a} (552) |
| | | 34.5 ± 2.9^{a} (867) | 0 (876) | 0 (876) | 1.2 ± 0.3^{a} (1014) | 53.6 ± 6.5^{a} (1302) | 23.0 ± 3.9^{a} (886) | 67.5 ± 8.5^{a} (918) | 32.2 ± 3.1^{a} (993) |
| 14 | Med or Neo Adv | 66.7± 4.7^{a} (954) | 37.4 ± 2.9^{a} (684) | 0^{a} (876) | 1.8 ± 1.0^{a} (678) | 70.1 ± 1.2^{a} (606) | 35.1 ± 3.2^{a} (486) | 72.5 ± 1.2^{a} (684) | 54.6 ± 5.3^{a} (678) |
| | | 10.8 ± 2.2^{a} (2406) | 4.9 ± 0.9^{a} (2280) | 0 (2280) | 0.9 ± 0.4^{a} (1620) | 81.1 ± 3.5 (2868) | 26.9 ±3.4^{a} (2217) | 35.3 ± 3.1^{a} (1944) | 13.3± 6.3^{a} (2262) |
| 42 | Med or Neo Adv | 91.5 + 2.4^{a} (2493) | 84.2 ± 10.2^{a} (2631) | 88.0 ± 2.5 (2838) | 0.1 ± 0.2 (2070) | 7.1 + 1.3^{a} (2493) | 7.2 ± 5.1^{a} (2787) | 5.8 ± 1.4 (2662) | 1.3 ± 1.9^{a} (2130) |
| | | 0.9 ± 0.5^{a} (1917) | 4.9 ± 3.1^{a} (2706) | 0.1 ± 0.2^{a} (2466) | 0.7 ± 0.7 (1713) | 14.4 ± 5.8^{a} (1917) | 4.4 ± 4.2 (2493) | 4.4 ± 1.3^{a} (2187) | 2.1 ± 1.3^{a} (1887) |

Animals (n=3) were euthanized at 0, 1, 3, 7, 14, 42 days after jugular vein transplantation. Tissue sections of vein grafts were stained with SMC contractile protein markers (SMA, Smoothlin, SM-MHC), TUNEL, VSCs markers (Sca-1, Sox17, NFM) and Ki67, and counterstained with DAPI. Total DAPI nuclei and different marker-positive nuclei were automatically counted by Velocity software (PerkinElmer, Waltham, MA, USA) in different layers of the vein grafts under 200 × magnification. At each time point, 3 sections per vein graft were analyzed. ^{a}p < 0.05 vs. the control normal vein (day 0).

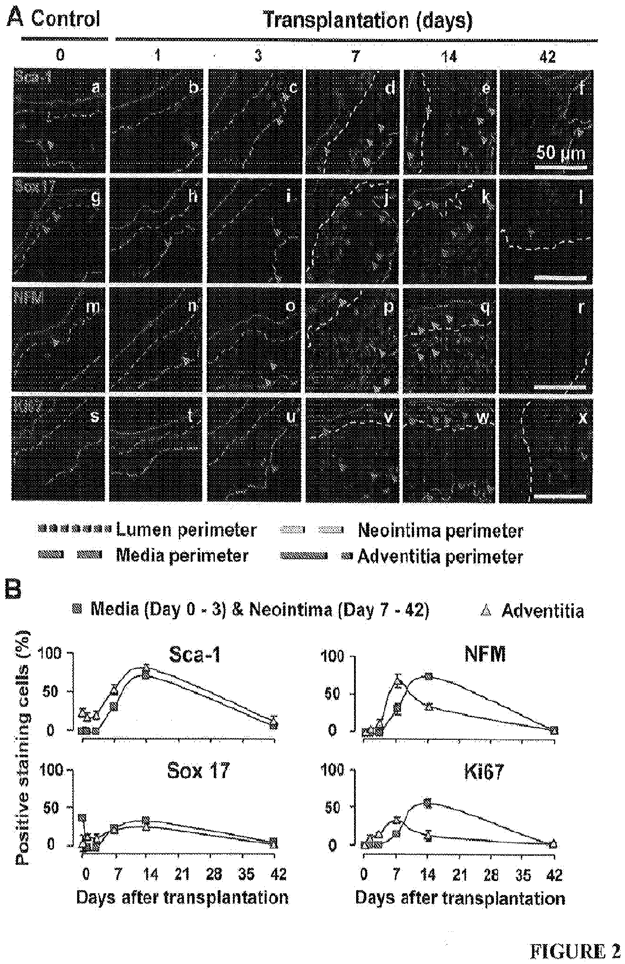

Considering that the synthetic SMCs in neointima of vein grafts originate predominantly from the local vessel wall, we postulated that resident VSCs are the major sources of synthetic SMCs leading to neointimal hyperplasia. Over the past decade, a growing body of evidence has revealed that various stem or progenitor cells are resident in the adult vessel wall, presumably participating in vascular repair and disease progression ^{8, 11, 27}. Notably, adventitia-derived Sca-1⁺ VSCs and media-derived Sca-1⁻, Sox10⁺, Sox17⁺, NFM⁺, and 5100β⁺ MVSCs are likely to be critical sources of synthetic SMCs in vascular lesion ^{8, 11}. Thus, we further analyzed a potential contribution of Sca-1⁺ cells and MVSCs to the generation of synthetic SMCs leading to neointima formation in vein isografts. Immunofluorescent staining showed that: 1) NFM⁺ and Sox17⁺ cells existed in the media, but Sca-1⁺ cells existed in the adventitia of normal jugular vein (Fig. 2A, panels a, -g, -m, and Fig. 2B). 2) The number of adventitial Sca-1⁺ cells was unchanged until 3 days after transplantation, then Sca-1⁺ cell number increased and these cells migrated into media, peaking at 14 days post-transplantation, and declined thereafter (Fig. 2A, panels -b to -f, and Fig. 2B). 3) Medial Sox17⁺ and NFM⁺ cells disappeared within 3 days, reappeared at 7 days, peaked at 14 days, and then declined, whereas the number of adventitial Sox17⁺, and NFM⁺ cells was increased at 7 days, peaked at 14 days, and then declined (Fig. 2A, panels h to -1, -n to -r, and B). 3) Locations of these stem cell marker positive cells moved toward neointima area, while neointimal hyperplasia progressed. 4) Adventitial Ki67⁺ cells appeared at 3 days and peaked at 7 days, whereas medial Ki67⁺ cells appeared at 7 days and peaked at 14 days. The number of Ki67⁺ cells in both media and adventitia declined 14 days after transplantation. The change pattern of Ki67⁺ cells is similar to that of the VSCs in vein grafts. The proportions of positive cells for each VSC marker and Ki67 in media/neointima and adventitia are summarized in Table 1. Taken together, these results indicate that medial VSCs, such as MVSCs, like mature SMCs in vein grafts die at the early stage after transplantation, while adventitial VSCs (Sca-1⁺ cells) survive or (Sox17⁺ and NFM⁺ cells) are activated. Thereafter, these surviving or activated VSCs proliferate and then probably differentiate into SMCs contributing to neointimal hyperplasia.

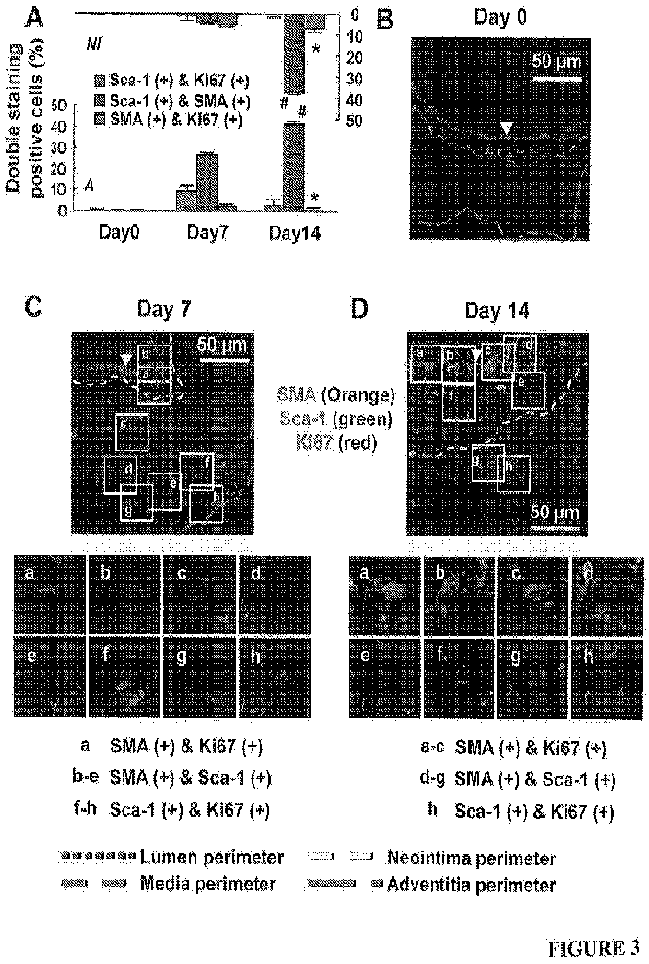

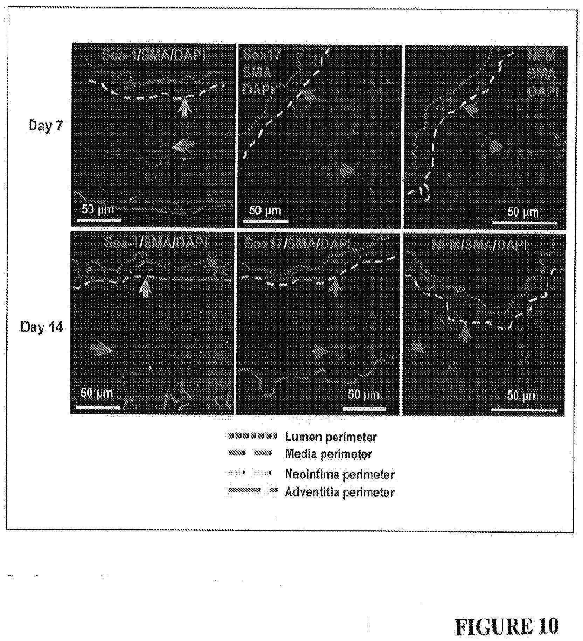

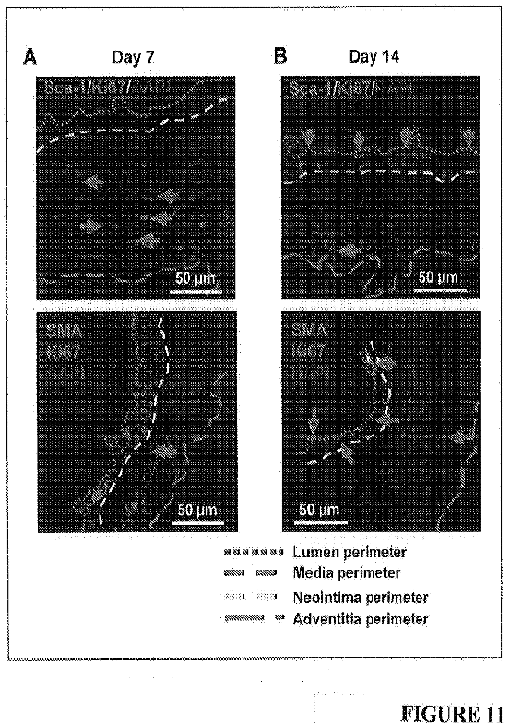

We noticed that the alternations in locations and numbers of Sca-1⁺, Sox17⁺, and NFM⁺ cells (Fig. 2) coincided with SMCs reappearance and number increase in media and neointima (Fig. 1), particularly at 7 and 14 days after transplantation. Accordingly, we hypothesized that some of these VSCs are the sources of the synthetic SMCs. To test this hypothesis, we performed double staining of VSC markers and SMA in vein grafts at 7 and 14 days. Of interest, Scal-1⁺ but not Sox17⁺ or NFM⁺ cells were positive for SMA (Fig. 10), suggesting that Sca-1⁺ cells may be the major source of the synthetic SMCs. In addition, Sca-1 and Ki67 double positive cells comprised the majority of cell population at 7 days, while proliferating SMCs (SMA and Ki67 double positive) were in the minority (Fig. 11A). Importantly, the majority of proliferating cells in neointima area were SMCs at 14 days, while the proliferating Sca-1⁺ cells were barely observed (Fig. 11B). These patterns suggest that Sca-1⁺ cells proliferate rapidly and subsequently differentiate into SMCs at 7 days. To support this notion, we conducted the immunofluorescence triple staining for SMA, Sca-1 and Ki67 and quantified proliferating Sca-1⁺ and/or SMA⁺ cells at 0, 7, and 14 days. As shown in Figure 3A, a decrease in proliferating Sca-1⁺ cells in adventitia was associated with an increase in proliferating SMCs in neointima from day 7 to day 14. Sca-1 and SMA double positive cells appeared in both adventitial and intimal layers at 7 and 14 days; however, the amount of these cells was increased from day 7 to day 14 in each layer (Fig. 3A). Interestingly, the double positive cells in adventitial layer exhibited a pattern migrating to neointimal layer from day 7 to day 14 (Fig. 3C and D). These results suggest that activated Sca-1 cells at 1 and 3 days proliferate and subsequently differentiate into SMCs at 7 and 14 days, thereby leading to neointima formation.

In summary, these results indicate that SMCs die while VSCs survive and proliferate in vein isografts at an early stage after transplantation, and then the VSCs, most likely Sca-1⁺ cells, differentiate into synthetic SMCs leading to the progression of neointimal hyperplasia.

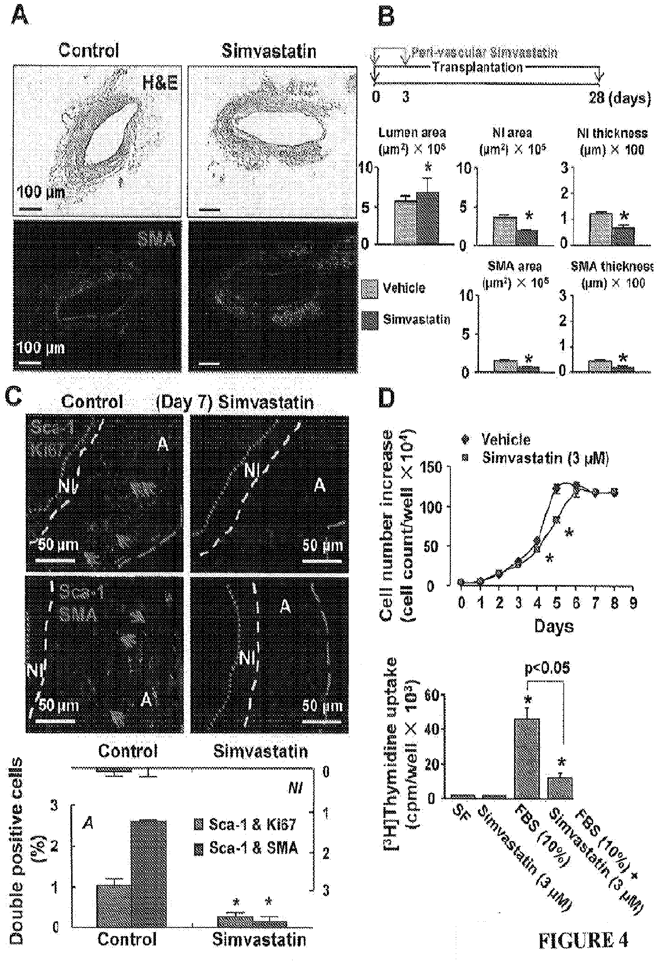

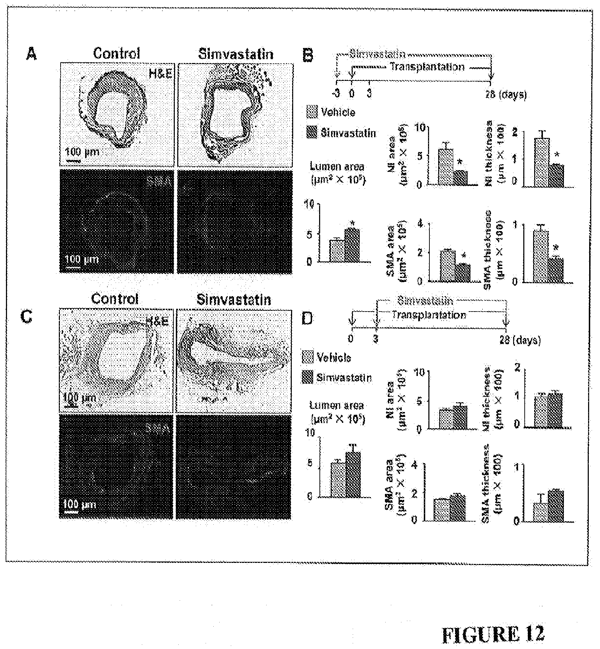

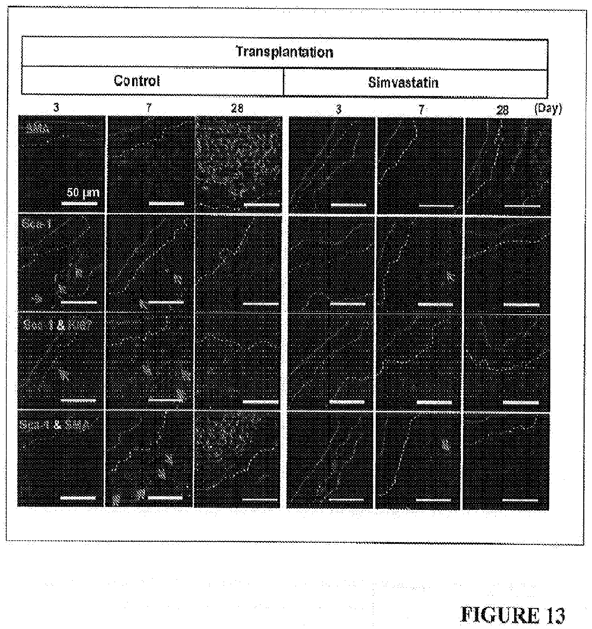

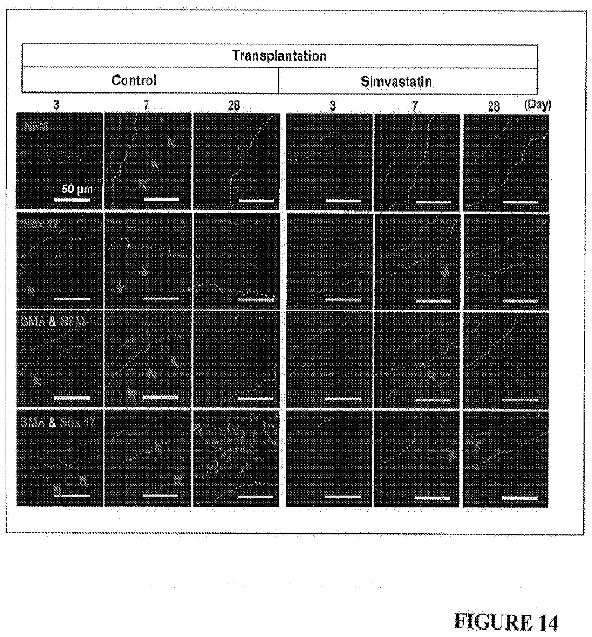

Next, we discovered that simvastatin inhibits neointimal hyperplasia primarily via suppressing the early activation of resident VSCs in vein isografts. To explore the pathophysiological relevance of early activation of resident VSCs in neointimal hyperplasia of vein grafts, we asked if resident VSCs could be potential drug targets of statins, the therapeutic efficacy of which has been well established for the suppression of neointimal hyperplasia of vein grafts in human and animal models ^{2, 15, 16}. We used simvastatin, which has been demonstrated to inhibit neointimal formation in a mouse model of vein graft ²⁸. We examined the efficacy of simvastatin which was administrated through three different routes in our model: 1) intragastric administration daily from 3 days before the transplantation until the endpoint as described elsewhere ²⁸; 2) intragastric administration daily starting 3 days after the transplantation until the endpoint; 3) peri-vascular delivery of simvastatin with pluronic-127 gel immediately after the transplantation, which could locally release the drug up to 3 days ¹⁷. Statin administration started from 3 days before transplantation suppressed neointimal formation in vein isografts 4 weeks after transplantation as described elsewhere ²⁸; however, statin administration started 3 days after the transplantation failed to inhibit the neointimal formation (Fig. 12A and B). These unexpected results suggest that simvastatin suppresses neointimal formation in vein grafts possibly via regulating the early activation of VSCs. Indeed, while the peri-vascular delivery of simvastatin immediately after the transplantation effectively inhibited neointimal formation as efficiently as the statin administration 3 days prior to transplantation (Fig. 4A and Fig. 12A), we observed that the peri-vascular statin treatment almost wiped out the double positive cells with Sca-1 and Ki67 or Sca-1 and SMA in vein grafts on day 3 and 7 as well as dramatically suppressed SMA⁺ cells and neointimal formation on day 28 after transplantation, compared to the vehicle-treated group (Fig. 13 and Fig. 4C). Moreover, the local statin treatment suppressed the increases in the number of NFM⁺ and Sox17⁺ cells at day 3 and 7 in vein isografts after transplantation, relative to the control (Fig. 14). On the other hand, we found that simvastatin inhibited Sca-1⁺ cell proliferation in vitro (Fig. 4D). These results indicate that simvastatin could suppress the early activation of resident VSCs and inhibit subsequent Sca-1⁺ cell differentiation into SMCs, which leads to neointimal formation.

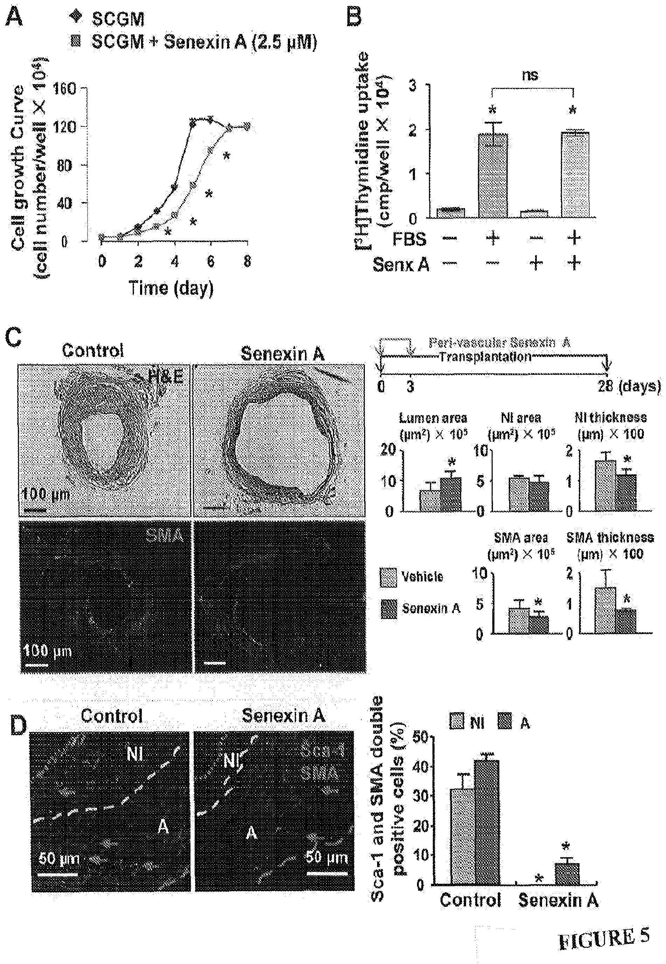

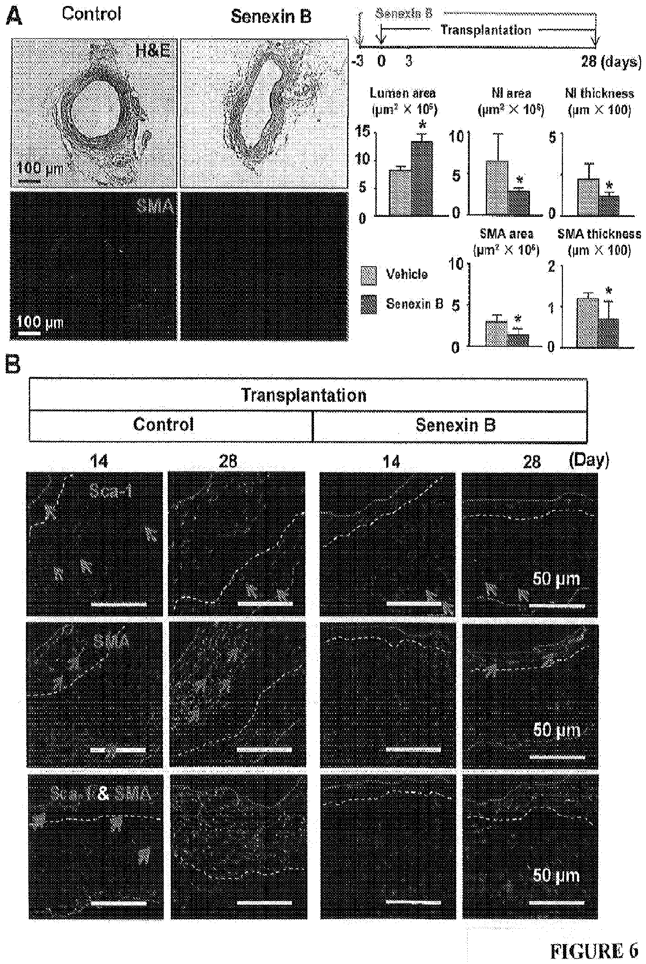

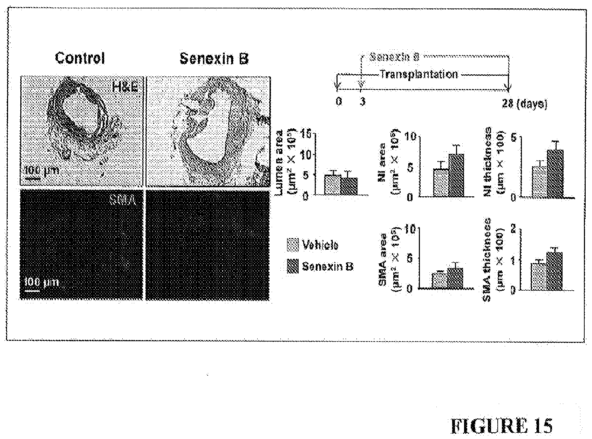

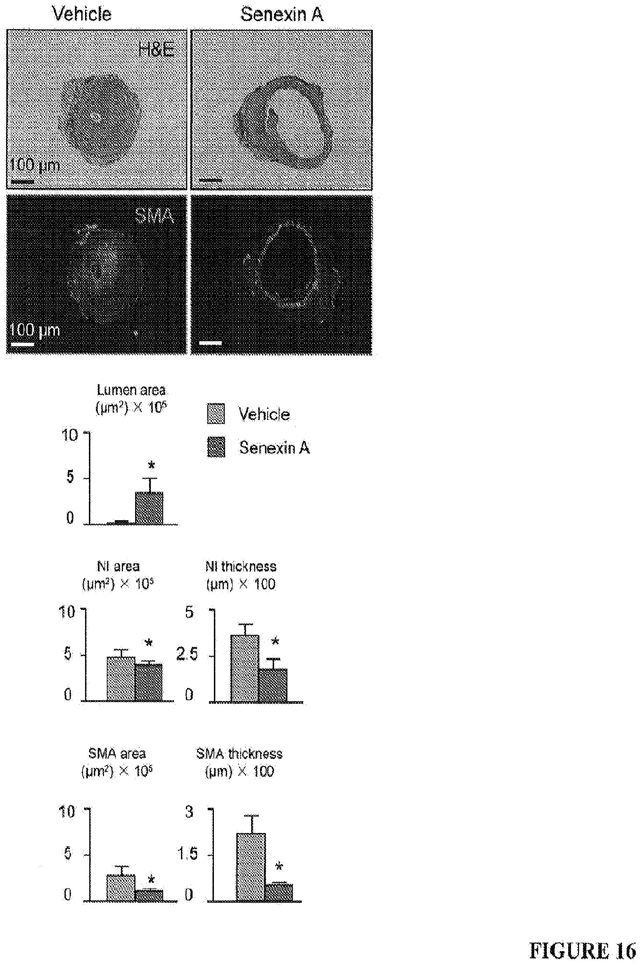

We next determined that Senexin A and B, CDK8/19 inhibitors, inhibit the proliferation of Sca-1⁺ cells but not VSMC and suppress neointimal formation of vein isografts. Senexin A ²⁰ and its derivative Senexin B, chemically optimized for increased potency and water solubility (US Patent Publication No. 20140038958) are highly selective inhibitors of CDK8 and its isoform CDK19, transcription-regulating kinases that promote the elongation of transcription of signal-activated genes ^{29, 30, 31}. CDK8 does not affect normal cell growth ^{32, 33} but regulates several transcriptional programs involved in carcinogenesis ³⁰. Two of the reported activities of CDK8 are potentially pertinent to VSC differentiation: the requirement for CDK8 in the embryonic stem cell phenotype ²¹ and potentiation of the transcriptional effects of TGF-β ²², a key mediator of VSC differentiation into SMCs ^{34, 35}. Thus, we hypothesized that CDK8 may play a key role in regulating VSC functions. Therefore we determined whether Senexin A or B differentially regulates the proliferation of VSCs and VSMCs. As shown in Fig. 5A and B, inhibition of CDK8 activity by Senexin A suppressed Sca-1⁺ cell proliferation without affecting rat aortic SMC (RASMC) growth, indicating a unique feature of CDK8 in regulating VSC proliferation. Taking advantage of Senexin-mediated selective suppression of VSC growth, we determined whether selective inactivation of VSCs at the early stage of remodeling in vein grafts could suppress the progression of neointimal hyperplasia. Similar to the results obtained with simvastatin, we observed that Senexin A or B suppressed neointimal formation when the drug treatment started 3 days prior to transplantation or via peri-vascular delivery immediately after transplantation (Fig. 5C and 6). In addition, Senexin A and B inhibited Sca-1⁺ cell differentiation into SMCs (Fig. 5C and 6). However, the inhibitory effect of Senexin B on neointimal formation in vein grafts was not observed when the drug treatment started 3 days after transplantation (Fig. 15). Of note, the inhibitory effect of peri-vascular delivery of Senexin A on neointimal formation in vein grafts lasted for 100 days after transplantation (Figure 16), although the release from Pluronic F-127 gel is locally maintained only for the initial 3 days after transplantation ¹⁷. Taken together, these results demonstrate that CDK8/19 inhibitors are capable of suppressing neointimal hyperplasia in vein grafts via inactivation of VSCs such as Sca-1⁺ cells.

Because the abnormal growth and accumulation of SMCs largely contribute to neointimal hyperplasia in vein grafts, most of the therapeutic approaches for the treatment of vein graft failure have focused on either direct or indirect inhibition of the SMC proliferation in vein grafts ^{2, 15, 36}. However, the 10-year vein graft failure rates (~50%) have remained largely unchanged over the last two decades. Thus it raises questions whether targeting SMCs is still a rational choice and if the optimal targets for the treatment of vein graft failure have been missed. In the present study, we have shown that mature SMCs die while resident VSCs survive and proliferate in vein grafts within 3 days after transplantation, and thereafter the VSC proliferation and SMC differentiation, SMC growth, and neointimal formation consecutively ensue. Surprisingly, we found that the inhibitory effect of simvastatin on neointimal formation in vein grafts is not likely due to its potential to inhibit SMC proliferation, but is largely dependent on its ability to suppress the early activation of resident VSCs. These results suggest that statins suppress neointimal hyperplasia primarily via inactivation of resident VSCs. In addition, we found that selective inactivation of resident VSCs via novel CDK8 inhibitors Senexin A and Senexin B at the early stage of vein graft remodeling inhibited the late neointimal formation in vein grafts. To the best of our knowledge, our findings uncover for the first time a causative link between the early activation of endogenous resident VSCs and the late neointimal formation in vein grafts *in vivo,* indicating that inactivation of resident VSCs at the early stage of vein graft remodeling represents a novel approach for the treatment of vein graft failure.

The majority of neointimal SMCs in vein grafts are derived from donors; i.e., the vein graft *per se,* and very few originate from the adjacent artery but not likely from the bone marrow ^{3, 4, 5, 6, 10}. Thus it is conceivable that the synthetic SMCs are predominantly derived from the dedifferentiation of mature vascular SMCs and/or SMC differentiation of resident VSCs in the vein graft *per se.* Because we observed that Sox17⁺ and NFM⁺ cells resided in media of normal veins and died along with media SMCs shortly after the vein transplantation, whereas adventitial Sca-1⁺ cells survived and differentiated into SMC in vein grafts, it is most likely that the adventitial Sca-1⁺ VSCs are the major source of synthetic SMCs in neointima of vein grafts. Indeed, this notion is strongly supported by our novel observations: 1) simvastatin suppressed neointimal formation in vein grafts primarily via inactivation of VSCs, including Sca-1⁺ cells; 2) CDK8 inhibitors Senexin A and Senexin B inhibited Sca-1⁺ cell growth and SMC differentiation but had no impact on SMC proliferation, and suppressed neointimal formation in vein grafts.

The essential link between the early inactivation of Sca-1⁺ cells and the suppression of neointimal hyperplasia in vein isografts of mice treated with simvastatin or CDK8 inhibitors is intriguing. Since simvastatin has a potential for selective suppression of abnormal stem cell expansion without affecting the self-renewal property of normal stem cells ³⁷, and also is able to suppress mesenchymal stem cell differentiation into SMCs ³⁸, while promoting a differentiated status of VSMCs ³⁹, it was not surprising that simvastatin suppressed Sca-1⁺ cell proliferation and SMC differentiation. In addition, it is possible that simvastatin may suppress the proliferation of abnormally activated VSCs while maintaining the normal VSC self-renewal for repair in the vein graft. However, the lack of efficacy of simvastatin treatment 3 day post-transplantation was unexpected, since the post-transplantation treatment with simvastatin, which started prior to the occurrence of extensive SMC proliferation in vein grafts, should have suppressed the neointimal hyperplasia, based on the well-described inhibitory effect of statins (including simvastatin) on VSMC proliferation ^{16, 28}. To reconcile these contrary observations, we proposed that the VSMCs used in previous statin studies ^{16, 28} may not be real SMCs but MVSCs as recently reported ¹¹, and thus emphasize the idea that primary cellular targets of statins for the treatment of neointimal hyperplasia in vein grafts are not VSMCs, but resident VSCs. On the other hand, the Senexin-induced suppression of VSC activation and neointimal hyperplasia could be due to its unique ability of selectively inactivating VSCs. Collectively, these results highlight an essential role of early activation of resident VSCs in neointimal hyperplasia of vein grafts as well as a therapeutic potential of targeting the early activated VSCs for the treatment of vein graft failure.

It should be noted that Sox17⁺ and NFM⁺ cells reappeared subsequently in the adventitia and may contribute to the progression of neointima formation in vein grafts; however, the precise source and role of these VSCs have not been addressed in the present study. One potential source may be the MVSCs, which are activated and migrate from the media of adjacent recipient arteries as recently reported ¹¹. However, the mechanism by which they lose the potency to differentiate into SMCs in the adventitia remains to be determined. Moreover, whether such a short-term inactivation of VSCs in the vein graft will lead to a long-term patency has not been explored in this study. The precise molecular mechanisms by which simvastatin and CDK8 inhibitors inactivate VSCs remain unknown. Further investigation of these issues in neointimal hyperplasia of vein grafts may provide valuable insights for a better understanding of VSCs in venous remodeling as well as suggest novel therapeutic interventions to treat vein graft failure.

The following examples are intended to further illustrate certain embodiments of the invention and are not to be construed as limiting its scope.

### Example 1

### Jugular vein transplantation

Male C57BL/6J mice were purchased from Vital River Laboratory Animal Technology Co. Ltd, Beijing, China, and housed under a 12:12 h light-dark cycle and given free access to food and water. All animal experiments were performed according to National Institutes of Health Guidelines for the Care and Use of Laboratory Animals and approved by the Institutional Animal Care and Usage Committees at Shandong University and the University of South Carolina, USA.The jugular vein transplantation was performed using different male C57BL/6J mice as donors and recipients. Briefly, 12-wk-old male C57BL/6J mice were anesthetized with pentobarbital sodium (50 mg/kg body weight, i.p.). The operation was performed under a dissecting microscope (SZ2-ILST, Olympus Corporation, Tokyo, Japan). The right common carotid artery of a male C57BL/6J mouse was mobilized and divided. A 1-mm cuff with a 1-mm handle (0.65 mm in diameter outside and 0.5 mm inside, F 800/200/100/200, Portex Ltd) was placed on both ends of the artery, and the ends were reverted over the cuff and ligated with an 8-0 silk ligature (130715, 130715, LingQiao). External jugular vein (1 cm) from a donor mouse was harvested and washed with saline solution, and grafted between the 2 ends of the carotid artery without changing the direction of blood flow. The ischemia time of vein segments was less than 15 min. The results are shown in Examples 7-9.

Fig. 7A shows representative H&E staining of mouse normal control jugular veins and jugular vein grafts 6 wks after transplantation. Dotted lines are the edges of lumen (L), media (M), neointima (NI), and adventitia (A) as indicated. Scale bars are 100 µm. Fig. 7B shows representative SMA immunofluorescence staining of mouse normal control jugular veins and jugular vein grafts 6 wks after transplantation. SMA is green. Nuclei labeled with 4',6-diamidino-2-phenylindole (DAPI) are blue. Dotted lines are indicated as above. Scale bars are 100 µm. Figure 7C shows the method of serial sectioning. The circle in blue indicates the proximal cuff. Sections began from the proximal cuff to the distant end without exposing the end cuff. One section (5 µm) from every ten sections (50 µm) was taken and numbered within a total collection of 100 sections (50 µm × 100 = 5 mm). Fig. 7D shows a scheme to depict structure of a vein graft. r indicates the lumen radius; green area indicates the SMA positive area; R₁ indicates the NI thickness + r (lumen radius); R₂ indicates SMA thickness + r. 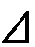 SMC indicates SMA positive area. 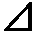 NI indicates neointima (NI) area.

Fig. 8A shows morphological analysis of serial H&E stained cross sections of 5-mm vein grafts from the proximal to distal end. Normal veins (n=4) and vein grafts (n=3) at 3 and 6 wks after surgery were analyzed. Lumen area, neointima (NI) area, and NI thickness were measured as described in Example 3 using Volocity software (PerkinElmer, Waltham, MA, USA). *p<0.05 vs. normal control (N). T; transplanted vein graft. Fig. 8B shows morphological analysis of serial SMA stained cross sections of 5-mm vein grafts from the proximal to distal end. Normal veins (n=4) and vein grafts (n=4) at 3 and 6 wks after surgery were analyzed. Lumen area, SMA area, and SMA thickness were measured as described in the Examples using Volocity software (PerkinElmer, Waltham, MA, USA). *p<0.05 vs. normal control (N). T; transplanted vein graft.

Fig. 9A shows representative H&E staining of vein cross sections at 0 day and 1, 3, 7, 14, 42 days after the transplantation. Dotted lines indicate edges of lumen, neointima, and adventitia. Fig. 9B shows schemes depicting main structures of vein grafts at different stages after the transplantation. Left panel: vein grafts at an early stage (0, 1, 3 days) are composed of media (green) and adventitia (orange). Right panel: vein grafts at late stage (7, 14, 28 days) are composed of neointima (yellow) and adventitia (orange).

### Example 2

### Histology

Mice at 0, 1, 3, 7, 14, 21 and 42 days after transplantation (four randomly chosen mice at each time point) were anesthetized with pentobarbital sodium (50 mg/kg body weight, i.p.) and euthanized by exsanguination. Blood vessels were fixed with 10% formalin under 100 mmHg (13.3kPa) pressure for 10 min and then washed with by 0.9% NaCl for 5 min. The vein grafts were harvested by cutting the transplanted segments from the native carotid arteries at the two cuff ends. The proximal ends of vein grafts were marked with 8-0 silk ligature. These grafts were further fixed with 4% phosphate-buffered formaldehyde at 4°C for 24 h, and then embedded in paraffin or OCT for sectioning. All the vein grafts were sectioned from the proximal end.

From the proximal end, the last section with the cuff tube was counted as #0. Serial cross-sections (5-µm thick per section) were then cut in 5-mm length without exposing the distal end with attached cuff tube. One section from every ten (50-µm length) was sampled and numbered with a final collection of 100 sample sections (50-µm × 100 = 5-mm) for each vein graft. Every five serial sections were mounted on one slide (50-µm × 5 = 250-µm), and a total of 20 slides was produced for each vein graft (250-µm × 20 = 5-mm).

Tissue sections were subjected to hematoxylin and eosin (H&E) or immunofluorescence staining as previously described⁴¹. Nuclei were counterstained with 4,6-diamidino-2-phenylindole (DAPI, cat#: C1002, Beyotime, Shanghai, China). Primary antibodies used were anti-SMA (Cat#: A5228SM, Sigma-Aldrich, St.Louis, MO, USA. 1:500 dilution), anti-SOX17 (Cat#: 09-038, Millipore, Temecula, CA, USA. 1:200 dilution), anti-NFM (Cat#: N4142, Sigma-Aldrich, 1:500 dilution), anti-Sca-1 (Cat#: 553333, BD, Franklin Lakes, NJ, USA. 1:200 dilution), anti-Smoothelin (Cat#: Sc-28562, Santa Cruz Biotechnology, Inc., Dallas, TX, USA. 1:200 dilution), anti-SM-MHC (Cat#: 5121-, Epitomics, Burlingame, CA, USA. 1:200 dilution) and anti-Ki67 (Cat#: Ab15580, Abcam, Cambridge, UK. 1:200 dilution). Corresponding fluorescent-conjugated IgG antibodies were used as secondary antibodies (Invitrogen). Fluorescent images were acquired by Nikon Eclipse Ti or UltraVIEW^{®}VOX confocal microscope and analyzed with the Volocity software (PerkinElmer, Waltham, MA, USA). In some images of immunofluorescence staining, the colors of biomarkers sometimes may not be the colors of the emitting light but the pseudo-colors generated by the Volocity software (PerkinElmer, Waltham, MA, USA).

### Example 3

### Morphological analysis

Lumen areas, neointima areas, and neointima thickness were measured as previously described ¹. In addition, considering the irregularly configured or even closed lumens of vein grafts during the tissue processing, lumen areas were also predicted by a formula: lumen area = πr² (Supplementary Fig. 1D). By tracking the perimeter length of a lumen which is minimally affected by the tissue processing, r, the radius of a lumen was calculated by a formula: r = perimeter of lumen length/2π. Similarly, the neointima thickness (ΔNI) was also calculated by a formula: ΔNI = R1 - r (Supplementary Figure 1D). Moreover, we enabled the SMA positive area (SMA area) to be calculated automatically by setting a fluorescent threshold for the immunofluorescent staining images of SMA using Volocity software (PerkinElmer, Waltham, MA, USA). Accordingly, SMA area thickness, which reflects SMC layer thickness, was calculated by a formula: ΔSMA = R2 - r (Supplementary Fig. S1D).

We reproduced the jugular vein transplantation model (n=3) as previously described 1. Initially, lumen and neointima areas were measured by tracking the perimeters of lumen and neointima. Neointima thickness was also quantified by measuring 4 regions of a section along a cross and recorded in micrometers. To get a solid conclusion, we analyzed 100 sections obtained by selecting the first of every 10 sections from each vein graft, reflecting the magnitude of neointima formation as well as the amount of neointimal SMCs in a 5-mm vein graft segment. The measured neointima area and thickness as well as SMA⁺ area and thickness were well correlated, showing a similar tendency. Of note, the indirectly calculated lumen area and neointima thickness were well consistent with the traditional measurements. Therefore, we determined neointima area and thickness, and lumen area by H&E staining while quantifying the amount of neointimal SMCs and the thickness of SMC layer by the SMA staining. The lumen area and neointima thickness were quantified indirectly by formulas: lumen area = πr² and neointima thickness (NI) = R2 - r (Supplementary Fig. S1D) respectively in the other experiments.

A further comparison between the results of analyzing ten sections (selecting the first of every 100 sections) and the results of analyzing 100 sections (selecting the first of every 10 sections) showed very similar tendencies (data not shown). Therefore, we determined neointima formation by analyzing ten sections selected from the first of every 100 sections in other experiments.

### Example 4

### Immunofluorescent microscopic analysisof Sca-1⁺ vascular stem cells (VSCs)

Aortic adventitial Sca-1⁺ cells were isolated and cultured as previously described²⁴. Briefly, the aortic arch and root together with a proportion of the heart from male 8-wk-old C57BL/6J mice were removed. Under a dissection microscope, adventitial tissues were carefully harvested. The adventitial tissues were cut into pieces (about 0.5 mm³) and explanted onto a 3.5 cm dish in a CO₂ incubator at 37°C for 2 h. The stem cell growth medium (CellGro SCGM, CellGenix, Germany) containing 10% FBS (Gibco, USA), 10 ng/ml leukemia inhibitory factor (Lif) (Millipore, Temecula, CA,USA) and 0.1 mmol/L 2-mercaptoethanol (Invitrogen, USA) was added and incubated for 5-7 days. Medium was changed every 2 days. To isolate Sca1⁺ cells, the cultured primary cells were applied to microbeads (Miltenyi Biotec, Germany) according to the manufacturer's instructions. Briefly, cells were dispersed with 0.25% trypsin and washed with running buffers (Miltenyi Biotec, Germany), and incubated with anti-Sca-1 immunomagnetic microbeads (Miltenyi Biotec, Germany). The cell suspension was added to a column equipped with a magnetic cell sorting system (MACS). After washing, Sca-1⁺ cells were collected. The purity and viability of isolated Sca-1⁺ cells were evaluated by immunostaining and trypan blue exclusion, respectively. Isolated cells in stem cell medium were seeded in a slide bottle and incubated in CO₂ incubator at 37°C.

Fig. 1A shows representative immunofluorescence staining of SMA, Smoothelin, SM-MHC, and TUNEL. SMA was labeled with orange (panels a to f), smoothelin with green (panels g to l), SM-MHC with purple (panels m to r), and TUNEL with red (panels s to x). Nuclei were labeled with DAPI (blue). The arrows indicate the representative positive cells. Dotted lines are the edges of lumen, media, neointima, and adventitia as indicated. Scale bars are 50 µm. Fig. 1B shows quantified percentages of the cells positive for SMC markers and TUNEL staining as indicated. Three cross-sections were randomly chosen at each time point per vein graft (n=3) and subjected to the immunofluorescent staining analysis.

Fig. 2A shows representative immunofluorescence staining of Sca-1, Sox 17, NFM, and Ki67. Sca-1 was labeled with green (panels a to f), Sox17 with red (panels g to l), NFM with orange (panels m to r), and Ki67with red (panels s to x). The nuclei were labeled with DAPI (blue). The arrows indicate the representative positive cells. Dotted lines are the edges of lumen, media, neointima, and adventitia as indicated. Scale bars are 50 µm. Fig. 2B shows quantified percentages of the cells positive with SMC markers and TUNEL staining as indicated. Three cross-sections were randomly chosen at each time point per vein graft (n=3) and subjected to the immunofluorescent staining analysis.

Fig. 3 shows results of tri-immunofluorescence staining of Sca-1, SMA, and Ki67 in cross-sections of jugular vein isografts on day 0, day 7, and day 14 after transplantation. Fig. 3A shows quantified proportions of the cells double positive with Sca-1 and Ki67, Sca-1 and SMA, and SMA and Ki67 in the adventitia and neointima. Three cross-sections were randomly chosen at each time point per vein graft and subjected to the immunofluorescent staining analysis. Tri-positive cells with SMA, Sca-1, and Ki67 were not observed (n=4). *p<0.05 vs. SMA and Ki67 double positive at 7 days; #p<0.05. Sca-1 and SMA double positive at 7 days. Figs. 3B-D show representative immunofluorescence staining of Sca-1 (green), SMA (orange), and Ki67 (red). Nuclei were labeled with DAPI (blue).

Fig. 10 shows representative double-immunofluorescence staining for SMA and VSCs markers Sca-1, Sox17, or NFM on tissue cross-sections of vein grafts (n=4) at 7 and 14 days after transplantation. For Sca-1 and SMA double staining, Sca-1 is labeled with green, and SMA is labeled with green. For Sox17 or NFM and SMA double staining, Sox17 or NFM is labeled with red, SMA is labeled with orange. The nuclei are labeled with DAPI (blue). The arrows indicate the representative positive cells. Dotted lines are the edges of lumen, media, neointima, and adventitia as indicated. Scale bars are 50 µm.

Fig. 11 shows representative double-immunofluorescence staining for Ki67 and Sca-1 or SMA on tissue cross-sections of vein grafts (n=4) at 7 and 14 days after transplantation. Ki67 is labeled with red, Sca-1 or SMA is labeled with green. The nuclei are labeled with DAPI (blue). The arrows indicate the representative double positive cells. Dotted lines are the edges of lumen, media, neointima, and adventitia as indicated. Scale bars are 50 µm.

### Example 5

### Cell culture

Rat aortic SMCs (RASMCs) were isolated from the thoracic aorta and cultured as described elsewhere⁴⁰. Enzymatically isolated SMCs from adult male Wistar rats were cultured in high glucose (4.5 g/L) Dulbecco's modified Eagle's medium (DMEM) (Cat#: 11995, Invitrogen, Grand Island, NY, USA) with 10% FBS (Invitrogen) in a CO₂ incubator at 37°C. Subculture was performed when the cells were grown to the 70~80% confluent state. We used the RASMCs at passage (P) 32 to do the [³H]thymidine uptake assay. The P5 Sca-1⁺ VSCs or P32 RASMCs were serum-starved for 24 h, and then cultured with full stem cell or regular growth medium with or without simvastatin for 24 h. [³H]thymidine (1 µCi/ml) were added during the last 4 h of culture. The radioactivity was measured as previously described⁴¹. Fig. 5A shows the effect of Senexin A on Sca-1⁺ cell growth, with a growth curve of mouse Sca-1⁺ cells cultured in stem cell growth medium with or without Senexin A (2.5 µM) for 8 days. n=4, *p<0.05 vs. vehicle control at each time point. Fig. 5B shows the effect of Senexin A on RASMC proliferation. Serum-starved RASMCs at passage 32 were treated with or without 10%FBS and Senexin (Senx A, 2.5 µM) for 24 h. n=4, *p<0.05 vs. control (-) in the same group.

P7 Sca-1⁺ VSCs (4 × 10⁴/well) were seeded in stem cell medium (SCGM + 10%FBS + 10 ng/ml Lif + 0.1 mmol/L 2-mercaptoethanol) with or without simvastatin (3 µmol/L) or Senexin A (2.5 µmol/L) in 6-well plates. Culture medium with or without simvastatin or Senexin A were changed every two days. The number of cells was counted daily for 8 days. Fig. 4D shows the effect of simvastatin on Sca-1⁺ cell growth. Upper panel; growth curve of mouse Sca-1⁺ cells cultured in stem cell growth medium with or without simvastatin (3 µM) for 8 days. n=4, *p<0.05 vs. vehicle control at each time point. Lower panel; [³H]thymidine uptake. n=4, ^{∗}p<0.05 vs. serum free (SF) control.

### Example 6

### Local drug treatment

Simvastatin (h20080360, Merk Sharp & Dohme Ltd, U.K.) and Senexin A were delivered locally follows: Simvastatin (30 µmol/L), Senexin A (3 µmol/L), or phosphate-buffered saline in 50 µL 20% Pluronic F-127 gel (Cat# : BCBH4538V, Sigma ) was delivered to the adventitia of grafted vessels, immediately after transplantation.

Fig. 4A shows the effect of the local simvastatin on neointima formation 4 weeks after transplantation. Left panel; representative H&E staining (upper panel) and SMA staining (lower panel). Fig. 4B shows quantified lumen areas, neointimal (IN) areas, NI thickness, and SMA positive areas and thickness. Ten cross-sections of each vein graft were subjected to the analysis (n=4). *p<0.05 vs. vehicle control. Fig 4C shows the effect of local simvastatin on Sca-1⁺ cell proliferation and SMC differentiation at 3 and 7 days. Upper panel; representative staining of Sca-1 (green), Ki67 (red) and SMA (orange) at 7 days. Lower panel; quantified proportions of double positive cells with Sca-1 and Ki67, Sca-1 and SMA, and SMA and Ki67 in the vein graft at 7 days. Three cross-sections were randomly chosen for each vein graft. n=4, *p<0.05 vs. control in the same group.

Fig. 13 shows the effect of perivascular simvastatin treatment on Sca-1⁺ VSC proliferation and SMC differentiation in jugular vein isografts. Representative immunofluorescence staining for SMA and Sca-1 on jugular vein isograft cross sections of vehicle and simvastatin (30 µmol/L)-treated groups (n=4) at 3, 7, 28 days after transplantation. Sca-1 is labeled with green, SMA is labeled with green and Ki67 is labeled with red. The nuclei are labeled with DAPI (blue). The arrows indicate the representative positive cells. Dotted lines are the edges of lumen, media, neointima, and adventitia as indicated. Scale bars are 50 µm.

Fig. 14 shows the effect of peri-vascular simvastatin treatment on NFM⁺ and Sox17⁺ VSC differentiation into SMCs in jugular vein isografts. Representative immunofluorescence staining for NFM and Sox17 and/or SMA on jugular vein isograft cross-sections of vehicle and simvastatin (30 µmol/L)-treated groups (n=4) at 3, 7 and 28 days after transplantation. NFM and Sox17 are labeled with red. SMA is labeled with green. The nuclei are labeled with DAPI (blue). The arrows indicate the representative positive cells. Dotted lines are the edges of lumen, media, neointima, and adventitia as indicated. Scale bars are 50 µm.

Fig. 5 shows the effect of peri-vascular delivery of Senexin A on Sca-1⁺ cell proliferation and SMC differentiation as well as neointima formation in the jugular vein isografts. Fig. 5C shows the effect of local Senexin A on neointima formation 4 weeks after transplantation. Left panel; representative H&E staining (upper panel) and SMA staining (lower panel). B, quantified lumen areas, neointima (IN) areas, NI thickness, and SMA positive areas and thickness. Ten cross-sections of each vein graft were subjected to the analysis (n=4). *p<0.05 vs. vehicle control. Fig. 5D shows the effect of local Senexin A on SMC differentiation at 7 days. Left panel; representative staining of Sca-1 (green) and SMA (orange) at 7 days. Lower panel; quantified proportions of double positive cells with Sca-1 and SMA in the vein graft at 7 days. Three cross-sections were randomly chosen for each vein graft. n=4, *p<0.05 vs. control in the same group.

### Example 7

### Systemic treatment

Fig. 12 shows the effect of simvastatin treatment on neointima formation in jugular vein isografts 4 wks after transplantation. Simvastatin (1.6 mg/kg/d) was administrated by intragastric gavage; (Figs. 12A and B) 3 days before transplantation or (Figs. 12C and D) 3 days after transplantation. Figs. 12 A and C show: Left panel; representative H&E staining (upper) and SMA staining (lower). Figs. 12B and D show quantified lumen areas, neointima (NI) areas, NI thickness, and SMA positive areas and thickness. SMA is labeled with red. Ten cross sections of each vein graft were analyzed. n=4, *p<0.05 vs. vehicle control.

Fig. 6 shows the effect of Senexin B treatment on neointimal formation and Sca-1⁺ VSC differentiation into SMCs in jugular vein isografts. Senexin B (20 mg/kg/d) was administered by intragastric gavages 3 days prior to transplantation. Vein grafts were harvested 4 wk after transplantation. Fig. 6A shows, Left panel; representative H&E staining (upper) and SMA staining (lower); Right panel; quantified lumen areas, neointima (NI) areas, NI thickness, and SMA positive areas and thickness. SMA is labeled with green. Ten cross-sections of each vein graft were analyzed. n=4, ^{∗}p<0.05 vs. vehicle control. Fig. 6B shows representative immunofluorescence staining for SMA and Sca-1 on jugular vein isograft cross-sections of vehicle and Senexin B-treated groups (n=4) at 14 and 28 days after transplantation. Sca-1 is labeled with green and SMA is labeled with brown. The nuclei are labeled with DAPI (blue). The arrows indicate the representative positive cells. Dotted lines are the edges of lumen, media, neointima, and adventitia as indicated. Scale bars are 50 µm.

Fig. 15 shows the effect of Senexin B treatment on neointimal formation in jugular vein isografts 4 wks after transplantation. Senexin B (20 mg/kg/d) was administered by intragastric gavages 3 days after transplantation. Left panel; representative H&E staining (upper) and SMA staining (lower). Right panel; quantified lumen areas, neointima (NI) areas, NI thickness, and SMA positive areas and thickness. SMA is labeled with red. Ten cross sections of each vein graft were analyzed. n=4, *p<0.05 vs. vehicle control.

### References

1. Harskamp RE, Lopes RD, Baisden CE, de Winter RJ, Alexander JH. Saphenous vein graft failure after coronary artery bypass surgery: pathophysiology, management, and future directions. Annals of surgery 257, 824-833 (2013).
2. Shukla N, Jeremy JY. Pathophysiology of saphenous vein graft failure: a brief overview of interventions. Current opinion in pharmacology 12, 114-120 (2012).
3. Hu Y, Mayr M, Metzler B, Erdel M, Davison F, Xu Q. Both donor and recipient origins of smooth muscle cells in vein graft atherosclerotic lesions. Circulation research 91, e13-20 (2002).
4. Xu Q, Zhang Z, Davison F, Hu Y. Circulating progenitor cells regenerate endothelium of vein graft atherosclerosis, which is diminished in ApoE-deficient mice. Circulation research 93, e76-86 (2003).
5. Zhang L, Freedman NJ, Brian L, Peppel K. Graft-extrinsic cells predominate in vein graft arterialization. Arteriosclerosis, thrombosis, and vascular biology 24, 470-476 (2004).
6. Jevon M, et al. Smooth muscle cells in porcine vein graft intimal hyperplasia are derived from the local vessel wall. Cardiovascular pathology : the official journal of the Society for Cardiovascular Pathology 20, e91-94 (2011).
7. Muto A, Model L, Ziegler K, Eghbalieh SD, Dardik A. Mechanisms of vein graft adaptation to the arterial circulation: insights into the neointimal algorithm and management strategies. Circulation journal : official journal of the Japanese Circulation Society 74, 1501-1512 (2010).
8. Torsney E, Xu Q. Resident vascular progenitor cells. Journal of molecular and cellular cardiology 50, 304-311 (2011).
9. Orlandi A, Bennett M. Progenitor cell-derived smooth muscle cells in vascular disease. Biochemical pharmacology 79, 1706-1713 (2010).
10. Daniel JM, Bielenberg W, Stieger P, Weinert S, Tillmanns H, Sedding DG. Time-course analysis on the differentiation of bone marrow-derived progenitor cells into smooth muscle cells during neointima formation. Arteriosclerosis, thrombosis, and vascular biology 30, 1890-1896 (2010).
11. Tang Z, et al. Differentiation of multipotent vascular stem cells contributes to vascular diseases. Nat Commun 3, 875 (2012).
12. Tang Z, Wang A, Wang D, Li S. Smooth muscle cells: to be or not to be? Response to Nguyen et Al. Circulation research 112, 23-26 (2013).
13. Hu Y, et al. Abundant progenitor cells in the adventitia contribute to atherosclerosis of vein grafts in ApoE-deficient mice. The Journal of clinical investigation 113, 1258-1265 (2004).
14. Chen Y, et al. Adventitial stem cells in vein grafts display multilineage potential that contributes to neointimal formation. Arteriosclerosis, thrombosis, and vascular biology 33, 1844-1851 (2013).
15. Margaritis M, Channon KM, Antoniades C. Statins and vein graft failure in coronary bypass surgery. Current opinion in pharmacology 12, 172-180 (2012).
16. Zhang L, et al. Local delivery of pravastatin inhibits intimal formation in a mouse vein graft model. Can J Cardiol 28, 750-757 (2012).
17. Torsney E, Mayr U, Zou Y, Thompson WD, Hu Y, Xu Q. Thrombosis and neointima formation in vein grafts are inhibited by locally applied aspirin through endothelial protection. Circulation research 94, 1466-1473 (2004).
18. Mayr M, Li C, Zou Y, Huemer U, Hu Y, Xu Q. Biomechanical stress-induced apoptosis in vein grafts involves p38 mitogen-activated protein kinases. FASEB journal : official publication of the Federation of American Societies for Experimental Biology 14, 261-270 (2000).
19. Westerband A, et al. Vein adaptation to arterialization in an experimental model. Journal of vascular surgery 33, 561-569 (2001).
20. Porter DC, et al. Cyclin-dependent kinase 8 mediates chemotherapy-induced tumor-promoting paracrine activities. Proceedings of the National Academy of Sciences of the United States of America 109, 13799-13804 (2012).
21. Adler AS, et al. CDK8 maintains tumor dedifferentiation and embryonic stem cell pluripotency. Cancer research 72, 2129-2139 (2012).
22. Alarcon C, et al. Nuclear CDKs drive Smad transcriptional activation and turnover in BMP and TGF-beta pathways. Cell 139, 757-769 (2009).
23. Thomas AC. Animal models for studying vein graft failure and therapeutic interventions. Current opinion in pharmacology 12, 121-126 (2012).
24. Zou Y, Dietrich H, Hu Y, Metzler B, Wick G, Xu Q. Mouse model of venous bypass graft arteriosclerosis. The American journal of pathology 153, 1301-1310 (1998).
25. Xu Q. Mouse models of vein grafts. Arteriosclerosis, thrombosis, and vascular biology 24, e185; author reply e185-187 (2004).
26. Xu Q. Mouse models of arteriosclerosis: from arterial injuries to vascular grafts. The American journal of pathology 165, 1-10 (2004).
27. Majesky MW, Dong XR, Hoglund V, Mahoney WM, Jr., Daum G. The adventitia: a dynamic interface containing resident progenitor cells. Arteriosclerosis, thrombosis, and vascular biology 31, 1530-1539 (2011).
28. Zhang L, Lu H, Huang J, Guan Y, Sun H. Simvastatin exerts favourable effects on neointimal formation in a mouse model of vein graft. Eur J Vasc Endovasc Surg 42, 393-399 (2011).
29. Galbraith MD, Donner AJ, Espinosa JM. CDK8: a positive regulator of transcription. Transcription 1, 4-12 (2010).
30. Xu W, Ji JY. Dysregulation of CDK8 and Cyclin C in tumorigenesis. Journal of genetics and genomics = Yi chuan xue bao 38, 439-452 (2011).
31. Galbraith MD, et al. HIF1A employs CDK8-mediator to stimulate RNAPII elongation in response to hypoxia. Cell 153, 1327-1339 (2013).
32. Firestein R, et al. CDK8 is a colorectal cancer oncogene that regulates beta-catenin activity. Nature 455, 547-551 (2008).
33. Westerling T, Kuuluvainen E, Makela TP. Cdk8 is essential for preimplantation mouse development. Molecular and cellular biology 27, 6177-6182 (2007).
34. Ross JJ, et al. Cytokine-induced differentiation of multipotent adult progenitor cells into functional smooth muscle cells. The Journal of clinical investigation 116, 3139-3149 (2006).
35. Cheung C, Bernardo AS, Trotter MW, Pedersen RA, Sinha S. Generation of human vascular smooth muscle subtypes provides insight into embryological origin-dependent disease susceptibility. Nature biotechnology 30, 165-173 (2012).
36. Desai M, Mirzay-Razzaz J, von Delft D, Sarkar S, Hamilton G, Seifalian AM. Inhibition of neointimal formation and hyperplasia in vein grafts by external stent/sheath. Vascular medicine 15, 287-297 (2010).
37. Xu H, Yang YJ, Yang T, Qian HY. Statins and stem cell modulation. Ageing research reviews 12, 1-7 (2013).
38. Kim KH, Kim YM, Lee MJ, Ko HC, Kim MB, Kim JH. Simvastatin inhibits sphingosylphosphorylcholine-induced differentiation of human mesenchymal stem cells into smooth muscle cells. Experimental & molecular medicine 44, 159-166 (2012).
39. Wada H, et al. Statins activate GATA-6 and induce differentiated vascular smooth muscle cells. Biochemical and biophysical research communications 374, 731-736 (2008).
40. Clempus RE, et al. Nox4 is required for maintenance of the differentiated vascular smooth muscle cell phenotype. Arteriosclerosis, thrombosis, and vascular biology 27, 42-48 (2007).
41. Li J, et al. Nrf2 protects against maladaptive cardiac responses to hemodynamic stress. Arteriosclerosis, thrombosis, and vascular biology 29, 1843-1850 (2009).

## Claims

1. One or more inhibitors of CDK8/19 for use in suppressing neointimal formation resulting from vascular surgery.

2. One or more inhibitors of CDK8/19 for use according to claim 1, wherein the vascular surgery is for occlusive vascular disease.

3. One or more inhibitors of CDK8/19 for use according to claim 2, wherein the occlusive vascular disease is atherosclerosis.

4. One or more inhibitors of CDK8/19 for use according to claim 1, wherein the vascular surgery is a vein graft implantation.

5. One or more inhibitors of CDK8/19 for use according to claim 4, wherein the vascular surgery is vein graft implantation in a patient via coronary artery bypass graft surgery or carotid artery bypass graft surgery.

6. One or more inhibitors of CDK8/19 for use according to claim 1, wherein the vascular surgery is percutaneous transluminal angioplasty.

7. One or more inhibitors of CDK8/19 for use according to claim 6, wherein the percutaneous transluminal angioplasty includes a stent emplacement.

8. One or more inhibitors of CDK8/19 for use according to claim 1, wherein the vascular surgery is creation of a native arteriovenous fistula.

9. One or more inhibitors of CDK8/19 for use according to claim 1, wherein the vascular surgery is implantation of a prosthetic hemodialysis arteriovenous graft.

10. One or more inhibitors of CDK8/19 for use according to claim 1, wherein the vascular surgery is an arterial graft transplant.

11. One or more inhibitors of CDK8/19 for use according to claim 10, wherein the arterial graft transplant is a coronary artery graft transplant.

12. One or more inhibitors of CDK8/19 for use according to claim 1, wherein the vascular surgery is orthotopic heart transplantation.

13. One or more inhibitors of CDK8/19 for use according to claim 7, wherein the one or more inhibitors of CDK8/19 is applied on a stent.

14. One or more inhibitors of CDK8/19 for use according to claim 1, wherein the one or more inhibitors of CDK8/19 is incorporated into an electrospun polymer on a stent and delivered on a dilatation balloon.

## Patentansprüche

1. Ein oder mehrere CDK8/19-Inhibitoren zur Verwendung bei der Unterdrückung der Neointimabildung infolge von Gefäßoperationen.

2. Ein oder mehrere CDK8/19-Inhibitoren zur Verwendung gemäß Anspruch 1, wobei die Gefäßoperation eine Gefäßverschlusskrankheit betrifft.

3. Ein oder mehrere CDK8/19-Inhibitoren zur Verwendung gemäß Anspruch 2, wobei die Gefäßverschlusskrankheit Atherosklerose ist.

4. Ein oder mehrere CDK8/19-Inhibitoren zur Verwendung gemäß Anspruch 1, wobei die Gefäßoperation eine Venentransplantation ist.

5. Ein oder mehrere CDK8/19-Inhibitoren zur Verwendung gemäß Anspruch 4, wobei die Gefäßoperation die Implantation eines Venentransplantats in einen Patienten mittels Koronararterien-Bypass-Transplantation oder Karotisarterien-Bypass-Transplantation ist.

6. Ein oder mehrere CDK8/19-Inhibitoren zur Verwendung gemäß Anspruch 1, wobei die Gefäßoperation eine perkutane transluminale Angioplastie ist.

7. Ein oder mehrere CDK8/19-Inhibitoren zur Verwendung gemäß Anspruch 6, wobei die perkutane transluminale Angioplastie eine Stentimplantation einschließt.

8. Ein oder mehrere CDK8/19-Inhibitoren zur Verwendung gemäß Anspruch 1, wobei die Gefäßoperation die Schaffung einer nativen arteriovenösen Fistel ist.

9. Ein oder mehrere CDK8/19-Inhibitoren zur Verwendung gemäß Anspruch 1, wobei die Gefäßoperation die Implantation eines prothetischen hämodialysearteriovenösen Transplantats ist.

10. Ein oder mehrere CDK8/19-Inhibitoren zur Verwendung gemäß Anspruch 1, wobei die Gefäßoperation eine arterielle Transplantation ist.

11. Ein oder mehrere CDK8/19-Inhibitoren zur Verwendung gemäß Anspruch 10, wobei die arterielle Transplantation eine Koronararterientransplantation ist.

12. Ein oder mehrere CDK8/19-Inhibitoren zur Verwendung gemäß Anspruch 1, wobei die Gefäßoperation eine orthotope Herztransplantation ist.

13. Ein oder mehrere CDK8/19-Inhibitoren zur Verwendung nach Anspruch 7, wobei der eine oder die mehreren CDK8/19-Inhibitoren auf einen Stent aufgebracht ist/sind.

14. Ein oder mehrere CDK8/19-Inhibitoren zur Verwendung gemäß Anspruch 1, wobei der eine oder die mehreren CDK8/19-Inhibitoren in ein elektrogesponnenes Polymer auf einem Stent eingearbeitet ist/sind und auf einen Dilatationsballon abgegeben wird/werden.

## Revendications

1. Un ou plusieurs inhibiteurs de CDK8/19 pour une utilisation dans la suppression de la formation néointimale résultant de la chirurgie vasculaire.

2. Un ou plusieurs inhibiteurs de CDK8/19 pour une utilisation selon la revendication 1, dans laquelle la chirurgie vasculaire est pour une maladie vasculaire occlusive.

3. Un ou plusieurs inhibiteurs de CDK8/19 pour une utilisation selon la revendication 2, dans laquelle la maladie vasculaire occlusive est l'athérosclérose.

4. Un ou plusieurs inhibiteurs de CDK8/19 pour une utilisation selon la revendication 1, dans laquelle la chirurgie vasculaire est une implantation d'un greffon veineux.

5. Un ou plusieurs inhibiteurs de CDK8/19 pour une utilisation selon la revendication 4, dans laquelle la chirurgie vasculaire est l'implantation d'un greffon veineux chez un patient via un pontage aorto-coronarien ou un pontage aorto-carotidien.

6. Un ou plusieurs inhibiteurs de CDK8/19 pour une utilisation selon la revendication 1, dans laquelle la chirurgie vasculaire est une angioplastie transluminale percutanée.

7. Un ou plusieurs inhibiteurs de CDK8/19 pour une utilisation selon la revendication 6, dans laquelle l'angioplastie transluminale percutanée comprend la mise en place d'un stent.

8. Un ou plusieurs inhibiteurs de CDK8/19 pour une utilisation selon la revendication 1, dans laquelle la chirurgie vasculaire est la création d'une fistule artério-veineuse native.

9. Un ou plusieurs inhibiteurs de CDK8/19 pour une utilisation selon la revendication 1, dans laquelle la chirurgie vasculaire est l'implantation d'un greffon artérioveineuse prothétique d'hémodialyse.

10. Un ou plusieurs inhibiteurs de CDK8/19 pour une utilisation selon la revendication 1, dans laquelle la chirurgie vasculaire est une transplantation de greffe artérielle.

11. Un ou plusieurs inhibiteurs de CDK8/19 pour une utilisation selon la revendication 10, dans laquelle la greffe artérielle est une greffe d'artère coronaire.

12. Un ou plusieurs inhibiteurs de CDK8/19 pour une utilisation selon la revendication 1, dans laquelle la chirurgie vasculaire est une transplantation cardiaque orthotopique.

13. Un ou plusieurs inhibiteurs de CDK8/19 pour une utilisation selon la revendication 7, dans laquelle le ou les inhibiteurs de CDK8/19 est/sont appliqués sur un stent.

14. Un ou plusieurs inhibiteurs de CDK8/19 pour une utilisation selon la revendication 1, dans laquelle le ou les inhibiteurs de CDK8/19 est/sont incorporés dans un polymère électrospongé sur un stent et délivrés sur un ballon de dilatation.
